# EUROPÄISCHE PATENTSCHRIFT

(11) **EP 3 260 193 B1**
(45) Veröffentlichungstag und Bekanntmachung des Hinweises auf die Patenterteilung: **17.10.2018**
(21) Anmeldenummer: 17174251.3
(22) Anmeldetag: 02.06.2017
(51) Int. Cl.: B01F 11/00, B01F 15/00, B01F 15/02, A61B 17/88

(54) **LAGER- UND MISCHVORRICHTUNG FÜR KNOCHENZEMENT MIT DRUCKPUMPE, UND VERFAHREN ZUR VERMISCHUNG EINES KNOCHENZEMENTS**
STORAGE AND MIXING DEVICE FOR BONE CEMENT WITH A PRESSURE PUMP AND METHOD FOR MIXING BONE CEMENT
DISPOSITIF DE MÉLANGE ET DE STOCKAGE POUR CIMENT OSSEUX À L'AIDE D'UNE POMPE DE REFOULEMENT ET PROCÉDÉ POUR MÉLANGER UN CIMENT OSSEUX

(30) Priorität: 08.06.2016 DE 102016110564
(43) Veröffentlichungstag der Anmeldung: 27.12.2017
(73) Patentinhaber: Heraeus Medical GmbH, 61273 Wehrheim (DE)
(72) Erfinder: VOGT, Sebastian, 99092 Erfurt (DE); KLUGE, Thomas, 56179 Vallendar (DE)
(74) Vertreter: Schultheiss & Sterzel Patentanwälte PartG mbB

(56) Entgegenhaltungen:
- EP-A2- 0 796 653
- WO-A1-2012/174670
- WO-A1-2014/205063

## Beschreibung

Die Erfindung betrifft eine Lager- und Mischvorrichtung für Zweikomponenten-Polymethylmethacrylat-Knochenzemente, die Lager- und Mischvorrichtung aufweisend eine Kartusche mit einem zylindrischen Innenraum.

Die Erfindung betrifft auch ein Verfahren zur Vermischung von Ausgangskomponenten eines Knochenzements, insbesondere eines Zweikomponenten-Polymethylmethacrylat-Knochenzements, mit einer solchen Lager- und Mischvorrichtung.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement, der üblicherweise als Knochenzementteig bezeichnet wird. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig. Aus der EP 0 796 653 A2 ist eine Vorrichtung gemäß dem Oberbegriff des Anspruchs 1 zur Herstellung von Knochenzement aus einem Knochenzementpulver und einer Monomerflüssigkeit in einer Kartusche bekannt, bei dem ein Vakuum mit einem anschließbaren Vakuumkolben gezogen wird. Nachteilig ist bei diesem System, dass nach dem Öffnen beziehungsweise Anschließen eines Monomerflüssigkeitsbehälters bereits Monomerflüssigkeit in das Knochenzementpulver eindringen und reagieren kann, bevor mit dem Vakuumkolben die restliche Monomerflüssigkeit in die Kartusche gesaugt wird. Dadurch können ausgehärtete Stücke Knochenzemente in dem Zementteig entstehen, wodurch die Anwendbarkeit des dadurch inhomogenen Knochenzementteigs erschwert wird und die mechanischen Eigenschaften des ausgehärteten Knochenzements negativ beeinflusst werden. Zudem ist durch die vielen notwendigen Schritte beim Anschließen der Teile der Vorrichtung und beim Herstellen des Vakuums die Anwendung der Vorrichtung relativ aufwendig und dadurch fehleranfällig.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

In der WO 00/35506 A1 wird eine Vorrichtung vorgeschlagen, bei der Polymethylmethacrylat-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei der Verwendung von Vakuummischsystemen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Ein interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für eine Mischstange oder ein Mischrohr nicht ohne weiteres dauerhaft vakuumdicht ist.

Die Aufgabe der Erfindung besteht also darin, die Nachteile des Stands der Technik zu überwinden. Insbesondere sollen die Nachteile der bekannten Vakuummischsysteme mit externer Vakuumquelle überwunden werden. Die Erfindung hat dabei unter anderem die Aufgabe, eine einfache geschlossene Lager- und Mischvorrichtung zu entwickeln, bei der Polymethylmethacrylat-Knochenzementpulver (Zementpulver) und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Lager- und Mischvorrichtung zusammengeführt und vermischt werden können, ohne dass beide Ausgangskomponenten mit dem medizinischen Anwender in Berührung kommen. Ein Kontakt des medizinischen Anwenders mit dem Polymethylmethacrylat-Knochenzementpulver und mit der Monomerflüssigkeit soll möglichst ausgeschlossen sein. Bei der zu entwickelnden Vorrichtung handelt es sich bevorzugt um ein Full-Prepacked-Mischsystem. Die Lager- und Mischvorrichtung soll so beschaffen sein, dass die Monomerflüssigkeit in das Polymethylmethacrylat-Knochenzementpulver ohne die Verwendung von durch Druckluft oder Kompressoren oder elektrisch angetriebenen externen Vakuumpumpen, transferiert werden kann. Wichtig ist ferner, dass die Lager- und Mischvorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Die Lager- und Mischvorrichtung soll ohne zusätzliche technische Ausrüstung autonom verwendungsfähig sein. Dabei soll die Lager- und Mischvorrichtung möglichst einfach und kostengünstig aufgebaut sein.

Die Lager- und Mischvorrichtung soll dahingehend vereinfacht sein, dass mit nur einem manuellen Bedienelement zuerst ein Monomerflüssigkeitsbehälter beziehungsweise eine Glasampulle oder ein Folienbeutel als Monomerflüssigkeitsbehälter geöffnet werden kann und dass danach, ohne Nutzung von extern erzeugtem Vakuum, ein Transfer der Monomerflüssigkeit in die Kartusche mit darin enthaltenem Zementpulver manuell vorgenommen werden kann. Eine Fehlbedienung soll konstruktiv möglichst ausgeschlossen werden.

Es soll ferner ein Verfahren bereitgestellt werden, das einen Monomerflüssigkeitstransfer und ein Mischen in Full-Prepacked-Mischsystemen ermöglicht und bei dem nach dem Öffnen des Monomerflüssigkeitsbehälters nur ein einziges Bedienelement bedient werden muss, um die Monomerflüssigkeit zu transferieren, einen Unterdruck in dem Mischraum (der Kartusche) zu erzeugen und mit dem Knochenzementpulver zu dem gewünschten Knochenzementteig zu mischen. Dabei soll die zu entwickelnde Lager- und Mischvorrichtung, hauptsächlich aus kostengünstigem Kunststoff gefertigt werden können.

Des Weiteren soll eine kostengünstig zu fertigende und zuverlässig funktionierende Lager- und Mischvorrichtung zum Mischen eines medizinischen Knochenzements und zur Lagerung der Ausgangskomponenten des Knochenzements sowie ein Verfahren zum Mischen des Knochenzements gefunden werden, bei dem eine möglichst einfache manuelle Bedienung zum Mischen der Ausgangskomponenten angewendet werden kann, möglichst ohne dass eine externe oder zusätzliche Energiequelle verwendet werden muss und ohne dass Lufteinschlüsse in dem Mischgut entstehen.

Die erste Ausgangskomponente des Polymethylmethacrylat-Knochenzements als Mischgut soll ein Pulver beziehungsweise Knochenzementpulver sein und die zweite Ausgangskomponente soll in Form einer Flüssigkeit, der Monomerflüssigkeit, vorliegen. Die beiden Ausgangskomponenten des Knochenzements sollen bevorzugt in dem Full-Prepacked-Mischsystem getrennt gelagert werden können und durch Anwendung der Lager- und Mischvorrichtung sicher zusammengeführt werden können.

Die Aufgaben der Erfindung werden gelöst durch eine Lager- und Mischvorrichtung für Zweikomponenten-Polymethylmethacrylat-Knochenzemente, die Lager- und Mischvorrichtung aufweisend
a) eine Kartusche mit einem zylindrischen Innenraum, wobei der zylindrische Innenraum an einer Vorderseite durch einen Kartuschenkopf mit einer geschlossenen Austragsöffnung begrenzt ist,
b) einen Kolben, der axial beweglich im zylindrischen Innenraum der Kartusche angeordnet ist und der von dem Kartuschenkopf beabstandet ist, wobei der Kolben umlaufend an der Innenwand des Innenraums anliegt, so dass der Kolben den Innenraum der Kartusche in zwei Bereiche trennt,
c) eine pulverförmige erste Ausgangskomponente des Knochenzements, die in dem Innenraum zwischen dem Kolben und dem Kartuschenkopf enthalten ist,
d) eine Durchführung, die im Kartuschenkopf oder in dem Zylindermantel der Kartusche zwischen dem Kolben und dem Kartuschenkopf angeordnet ist, wobei die Durchführung mit einer Fluidleitung verbunden ist,
e) eine Aufnahme für eine Monomerflüssigkeit, wobei die Fluidleitung die Durchführung mit der Aufnahme flüssigkeitsdurchlässig verbindet, wobei die Monomerflüssigkeit als zweite Ausgangskomponente des Knochenzements in der Aufnahme enthalten ist oder in die Aufnahme einfüllbar oder einleitbar ist,
f) eine Ausdrückvorrichtung, die in die Aufnahme hinein zu drücken ist, so dass Monomerflüssigkeit aus der Aufnahme in die Fluidleitung hinein drückbar ist,
g) wobei der Kolben von einer der Vorderseite gegenüberliegenden Rückseite des zylindrischen Innenraums beabstandet ist, so dass durch eine Bewegung des Kolbens in Richtung der Rückseite die Ausdrückvorrichtung in die Aufnahme hinein zu drücken ist und ein Unterdruck in dem Innenraum der Kartusche zwischen dem Kolben und dem Kartuschenkopf erzeugbar ist.

Wenn der Unterdruck in dem Innenraum der Kartusche zwischen dem Kolben und dem Kartuschenkopf durch die Bewegung des Kolbens erzeugt ist, wird dieser Unterdruck durch die Durchführung und in die Fluidleitung geleitet.

Als Ausdrückvorrichtungen wird bevorzugt ein Pumpkolben verwendet. Es kann auch eine elastische Membran als Ausdrückvorrichtung verwendet werden, wobei die elastische Membran sich in die Aufnahme ausdehnt (und dadurch sich im Sinne der vorliegenden Erfindung in die Aufnahme hinein bewegt beziehungsweise in die Aufnahme hinein gedrückt wird) und so die Monomerflüssigkeit aus der Aufnahme in die Fluidleitung drückt.

Bevorzugt ist zwischen dem Kolben und der pulverförmigen ersten Ausgangskomponenten kein weiteres Bauteil vorgesehen.

Bevorzugt kann vorgesehen sein, dass die erste Ausgangskomponente ein Zementpulver ist.

Ferner kann bevorzugt vorgesehen sein, dass der Kolben den Innenraum der Kartusche gasdicht in zwei Bereiche trennt. Dadurch kann sichergestellt werden, dass durch die Bewegung des Kolbens in Richtung der Rückseite ein Unterdruck im Innenraum der Kartusche erzeugt und zumindest bis zum Ende des Mischvorgangs gehalten werden kann.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die begrenzende Innenwand des Innenraums kann also ein Zylinder mit beliebiger Grundfläche sein und ebenso kann gegebenenfalls der Mantel der Kartusche ein Zylinder mit beliebiger Grundfläche sein, das heißt auch mit nicht kreisförmiger oder nicht runder Grundfläche. Erfindungsgemäß wird jedoch eine zylindrische Geometrie mit rotationssymmetrischer und insbesondere kreisrunder Grundfläche für den Innenraum der ersten Kartusche bevorzugt, da diese am einfachsten zu fertigen sind und der Kolben dann weniger leicht im Innenraum verkannten kann, wenn er axial im Innenraum bewegt wird. Zudem werden mögliche Undichtigkeiten zwischen der Innenwand des Innenraums und dem Kolben während der Bewegung des Kolbens unwahrscheinlicher.

Dass der Kolben im zylindrischen Innenraum der Kartusche axial beweglich ist bedeutet, dass der Kolben entlang der Zylinderachse des zylindrischen Innenraums axial beweglich ist.

Es kann erfindungsgemäß vorgesehen sein, dass der Kolben ein Austragskolben ist, mit dem der gemischte Knochenzementteig durch Vortreiben des Austragskolbens in Richtung Kartuschenkopf aus der Kartusche durch die Austragsöffnung oder durch das Austragsrohr oder den hohlen Mischstab auszupressen ist.

Die Rückseite des Innenraums bildet auch die Rückseite der Kartusche.

Bei erfindungsgemäßen Lager- und Mischvorrichtungen kann vorgesehen sein, dass der Kolben mit einem Stab oder mit einem anderen Kraftübertragungsmittel in dem Innenraum der Kartusche manuell in Richtung der Rückseite des Innenraums zu drücken oder zu ziehen ist, wobei vorzugsweise an dem Stab oder an dem Kraftübertragungsmittel ein Betätigungsmittel oder ein Griff zum Bedienen des Stabs oder des Kraftübertragungsmittels befestigt ist.

Hiermit kann der Unterdruck leicht und unmittelbar manuell erzeugt werden und die Ausdrückvorrichtung und damit die Monomerflüssigkeit einfach manuell angetrieben werden, was einerseits die Gefahr möglicher Fehlfunktionen reduziert und andererseits komplexe und kostenintensive Bauteile wie Motoren, Energiespeicher oder Steuerungen vermieden werden. Zudem kann so eine einfache und sichere Bedienbarkeit der Lager- und Mischvorrichtung gewährleistet werden.

Ferner kann vorgesehen sein, dass im Kartuschenkopf, in der Durchführung und/oder in der Fluidleitung ein Filter, insbesondere ein Porenfilter, angeordnet ist, wobei der Filter für die Monomerflüssigkeit durchlässig und für die pulverförmige erste Ausgangskomponente undurchlässig ist.

Hiermit wird verhindert, dass Zementpulver beziehungsweise die pulverförmige erste Ausgangskomponente in die Fluidleitung bis zur Monomerflüssigkeit vordringen kann, dort mit der Monomerflüssigkeit vorzeitig reagiert, fest beziehungsweise gel-artig wird und dadurch die Fluidleitung verschließt. Hierzu ist der Filter vorzugsweise im Kartuschenkopf, in der Durchführung oder in der Fluidleitung im Bereich der Durchführung angeordnet.

Bevorzugt kann auch vorgesehen sein, dass der Kolben von einer der Vorderseite gegenüberliegenden Rückseite des zylindrischen Innenraums derart weit beabstandet ist, dass mit dem Unterdruck die Monomerflüssigkeit aus der Fluidleitung in den Innenraum der Kartusche zu saugen ist.

Hiermit wird erreicht, dass der Hub des Kolbens auch dazu ausreicht, die Monomerflüssigkeit mit Hilfe des im Innenraum der Kartusche entstehenden Unterdrucks durch die Fluidleitung in den Innenraum der Kartusche zu saugen, zusätzlich zu dem Transport, der durch die Bewegung der Ausdrückvorrichtung in die Aufnahme erfolgt.

Es kann dazu ferner vorgesehen sein, dass der Kolben derart (beziehungsweise derart weit) von der Rückseite des zylindrischen Innenraums beabstandet ist, dass der Hubraum des Kolbens bis zu der Rückseite zumindest genauso groß ist, wie das Volumen der einzusaugenden Monomerflüssigkeit, vorzugsweise zumindest genauso groß ist, wie das Volumen der Fluidleitung und der Aufnahme.

Mit einer Weiterbildung der vorliegenden Erfindung wird vorgeschlagen, dass die Lager- und Mischvorrichtung einen von der Kartusche und von der Aufnahme separaten Behälter aufweist, in dem die Monomerflüssigkeit enthalten ist, wobei der Behälter mit der Aufnahme verbunden ist, insbesondere über eine Öffnung verbunden ist, wobei vorzugsweise in dem Behälter eine geschlossene Glasampulle oder ein Folienbeutel enthaltend die Monomerflüssigkeit angeordnet oder anzuordnen ist, wobei die Glasampulle innerhalb des Behälters aufbrechbar ist oder der Folienbeutel innerhalb des Behälters aufzustechen oder aufzureißen ist.

Hierdurch wird ein sogenanntes Full-Prepacked-Mischsystem bereitgestellt, bei dem alle Ausgangskomponenten, das heißt die beiden Ausgangskomponenten (Zementpulver als erste pulverförmige Ausgangskomponente und Monomerflüssigkeit als zweite Ausgangskomponente) bereits in der Lager- und Mischvorrichtung enthalten sind, darin auch über längere Zeiträume gelagert werden können und innerhalb der Lager- und Mischvorrichtung gemischt werden können.

Bei solchen Lager- und Mischvorrichtungen kann vorgesehen sein, dass die Kartusche, die Aufnahme, der separate Behälter und die Fluidleitung mit einem gemeinsamen Standfuß verbunden sind, wobei die Aufnahme, der Behälter und die Fluidleitung fest mit dem Standfuß verbunden sind und die Kartusche lösbar mit dem Standfuß verbunden ist, bevorzugt die Kartusche über ein Gewinde an den Standfuß geschraubt ist oder über eine Rastung mit dem Standfuß verbunden ist.

Hiermit wird die Anwendung der Lager- und Mischvorrichtung vereinfacht. In Operationssälen ist meist eine ebene Unterlage, wie beispielsweise ein Tisch, vorhanden, so dass die Lager- und Mischvorrichtung mit Hilfe des Standfußes dort aufgestellt und angewendet werden kann, ohne dass die Lager- und Mischvorrichtung in der Hand gehalten werden müsste. Hierdurch wird die Anwendung der Lager- und Mischvorrichtung weiter vereinfacht.

Des Weiteren kann vorgesehen sein, dass in dem separaten Behälter ein Monomerflüssigkeitsbehälter enthalten ist, insbesondere ein Folienbeutel oder eine Glasampulle enthalten ist, der oder die innerhalb des separaten Behälters zu öffnen ist, so dass die Monomerflüssigkeit aus dem geöffneten Monomerflüssigkeitsbehälter in die Aufnahme fließt, wobei vorzugsweise am Behälter eine von außen bedienbare Öffnungsvorrichtung zum Öffnen des Monomerflüssigkeitsbehälters angeordnet ist.

Hierdurch wird ein sogenanntes Full-Prepacked-Mischsystem bereitgestellt, bei dem alle Ausgangskomponenten, das heißt die beiden Ausgangskomponenten (Zementpulver als erste pulverförmige Ausgangskomponente und Monomerflüssigkeit als zweite Ausgangskomponente) bereits in der Lager- und Mischvorrichtung enthalten sind, darin gelagert werden können und innerhalb der Lager- und Mischvorrichtung gemischt werden können. Die Glasampulle oder der Folienbeutel, der vorzugsweise mit Aluminium beschichtet ist, sind zur langfristigen Lagerung der Monomerflüssigkeit als zweiter Ausgangskomponente zur Herstellung des PMMA-Knochenzementteigs besonders gut geeignet. Die Anwendung der Lager- und Mischvorrichtung wird so vereinfacht und erleichtert.

Damit die Monomerflüssigkeit zunächst ohne zusätzliche Krafteinwirkung in die Aufnahme fließen kann, muss die Lager- und Mischvorrichtung bestimmungsgemäß aufgestellt werden, damit die Gravitation die gewünschte Flussrichtung bewirkt. Demzufolge sind die im Rahmen der vorliegenden Erfindung verwendeten Begriffe oben und unten sowie oberhalb und unterhalb immer in Bezug auf die bestimmungsgemäße Aufstellung der Lager- und Mischvorrichtung bezogen.

Es kann auch ein Monomerflüssigkeitsbehälter mit mehreren Kammern zur Aufbewahrung der Monomerflüssigkeit verwendet werden. Unter einem Monomerflüssigkeitsbehälter wird im Rahmen der vorliegenden Erfindung also auch eine Vielzahl von separaten einzelnen Teil-Monomerflüssigkeitsbehältern verstanden, die in den separaten Behälter eingebracht werden können oder sind und die mit der Öffnungsvorrichtung geöffnet werden können.

Es kann vorgesehen sein, dass in der Zuleitung zur Aufnahme oder auch in oder an der Fluidleitung zur Kartusche ein Sieb oder ein Filter angeordnet ist, mit dem Bruchstücke oder Fetzen des geöffneten Monomerflüssigkeitsbehälters zurückgehalten werden können. Bevorzugt befindet sich das Sieb oder der Filter in dem Behälter direkt unterhalb des Monomerflüssigkeitsbehälters.

Mit einer Weiterbildung der Erfindung zur Ausnutzung der Gravitation als Antrieb für den Fluss der Monomerflüssigkeit in die Fluidleitung wird vorgeschlagen, dass der separate Behälter für den Monomerflüssigkeitsbehälter oberhalb der Aufnahme angeordnet ist. Dadurch kann die Monomerflüssigkeit aus dem Monomerflüssigkeitsbehälter nach dem Öffnen des Monomerflüssigkeitsbehälters aufgrund der Gravitation nach unten in die Aufnahme fließen.

Gemäß einer bevorzugten Weiterbildung der Erfindung kann vorgesehen sein, dass in dem Innenraum der Kartusche zwischen dem Kolben und dem Kartuschenkopf eine Mischeinrichtung angeordnet ist, mit der die erste Ausgangskomponente mit der Monomerflüssigkeit im Innenraum der Kartusche zu mischen ist.

Hiermit wird erreicht, dass die erste Ausgangskomponente mit der Monomerflüssigkeit im Innenraum der Kartusche gut durchmischt werden kann, um einen möglichst homogenen Knochenzementteig zu erzeugen. Bevorzugt kann die Mischeinrichtung manuell von außen bedient werden.

Dabei kann vorgesehen sein, dass die Mischeinrichtung über einen Mischstab von außerhalb der Kartusche bedienbar ist, wobei der Mischstab gasdicht durch den Kartuschenkopf geführt ist sowie drehbar und in axialer Richtung beweglich ist, vorzugsweise der Mischstab durch die Austragsöffnung geführt ist.

Hiermit kann die Mischeinrichtung einfach und kraftvoll von außerhalb der Lager- und Mischvorrichtung manuell bedient werden. Zudem sind durch den Widerstand gegen die Bewegung der Mischeinrichtung die Konsistenz des Knochenzementteigs und damit die Einsatzfähigkeit des Knochenzementteigs durch den Anwender gut abzuschätzen.

Es kann erfindungsgemäß vorgesehen sein, dass im Bereich des Kartuschenkopfs außen an der Kartusche ein Befestigungsmittel, insbesondere ein Außengewinde, vorgesehen ist. Daran kann ein separates Austragsrohr befestigt werden. Bevorzugt wird jedoch ein hohler rohrförmiger Mischstab verwendet, mit dem die Mischeinrichtung bedienbar ist und mit dem der Kolben in dem Innenraum in Richtung der Rückseite des Innenraums gedrückt werden kann, um den Unterdruck im Innenraum zu erzeugen, wobei der rohrförmige Mischstab nach Entfernen eines Verschlusses aus dem rohrförmigen Mischstab als Austragsrohr verwendet werden kann. Dann ist kein Außengewinde mehr notwendig, wobei das Außengewinde auch zur Befestigung der Kartusche in einer Austragsvorrichtung beziehungsweise Auspressvorrichtung zum Vortreiben des Kolbens in Richtung Kartuschenkopf verwendet werden kann, um den fertig gemischten Knochenzementteig auszupressen und zu applizieren. Um die Austragsöffnung zu schließen ist dazu im Inneren des hohlen Mischstabs ein herausziehbarer Kern angeordnet, der den rohrförmigen Mischstab nach außen verschließt.

Ferner kann vorgesehen sein, dass der Kolben mit dem Mischstab in Richtung der Rückseite des Innenraums der Kartusche zu drücken ist.

Hiermit kann der Kolben mit der gleichen Vorrichtung, die auch zum Antreiben der Mischeinrichtung verwendet wird, nämlich mit dem Mischstab, angetrieben werden, um die Ausdrückvorrichtung in die Aufnahme zu drücken und um den Unterdruck im Innenraum der Kartusche zu erzeugen. Hiermit wird für beide beziehungsweise für alle drei Funktionen nur eine gemeinsame Vorrichtung benötigt und die Lager- und Mischvorrichtung benötigt nur eine abzudichtende Durchführung hierzu.

Des Weiteren kann vorgesehen sein, dass der Mischstab ein Austragsrohr ist, in dem ein manuell entfernbarer Kern angeordnet ist, so dass der gemischte Knochenzementteig aus dem Innenraum der Kartusche durch das Austragsrohr ohne den Kern auszutreiben ist.

Hierzu wird das Austragsrohr, vorzugsweise zuvor, bis zu einem Anschlag aus dem Innenraum der Kartusche herausgezogen. Durch die Verwendung des Mischstabs als Austragsrohr wird kein zusätzliches oder separates Austragsrohr benötigt, das an der Lager- und Mischvorrichtung zu befestigen wäre. Der Mischstab ist zu diesem Zweck gegen den Kartuschenkopf abgedichtet und axial beweglich und drehbar in der Austragsöffnung beziehungsweise durch die Austragsöffnung geführt.

Alternativ kann vorgesehen sein, dass die Lager- und Mischvorrichtung ein separates Austragsrohr mit einem statischen Mischer aufweist, das an der Kartusche zu befestigen ist, vorzugsweise an dem Befestigungsmittel an der Kartusche zu befestigen ist, wobei besonders bevorzugt an dem Austragsrohr ein zum Außengewinde an der Kartusche passendes Innengewinde vorgesehen ist und/oder Elemente eines Bajonettverschlusses und/oder Rastelemente eines Rastverschlusses vorgesehen sind.

Es wird mit der vorliegenden Erfindung auch vorgeschlagen, dass im Innenraum der Kartusche an der Rückseite ein Anschlag vorgesehen ist, der die Bewegung des Kolbens in Richtung Rückseite begrenzt.

Hiermit wird verhindert, dass der Kolben über die Rückseite des Innenraums hinaus verschoben werden kann und ungewollt Knochenzementteig nach außen austritt.

Es kann auch vorgesehen sein, dass der Kolben mindestens ein Rastelement besitzt, das mit einer Gegenrastung an der Innenseite der Kartusche im Bereich der Rückseite des Innenraums lösbar zu rasten ist.

Hierdurch wird der Kolben, wenn er bis zur Rückseite bewegt wurde, in Position gehalten. Dadurch werden ein ungewolltes Bewegen des Kolbens während der Mischung des Inhalts des Innenraums und eine Bewegung des Kolbens über die Rastposition hinaus vermieden. Um den fertig gemischten Knochenzementteig auszutragen, kann die Rastung einfach gelöst werden.

Bei erfindungsgemäßen Lager- und Mischvorrichtungen kann vorgesehen sein, dass die Kartusche, die Aufnahme, der Kartuschenkopf und der Kolben, vorzugsweise auch ein Pumpkolben als Ausdrückvorrichtung, aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Bevorzugte Lager- und Mischvorrichtungen können sich auch dadurch auszeichnen, dass im Kartuschenkopf eine gasdurchlässige Öffnung, insbesondere eine verschließbare gasdurchlässige Öffnung, angeordnet ist, wobei eine für Gase durchlässige und für Feststoffpartikel undurchlässige poröse Scheibe zwischen der ersten Ausgangskomponente und der Öffnung angeordnet ist, wobei bevorzugt die poröse Scheibe im Kartuschenkopf angeordnet ist.

Damit kann eine Sterilisierung der Lager- und Mischvorrichtung auch im Innenraum der Kartusche mit Hilfe eines sterilisierenden Gases, wie beispielsweise Ethylenoxid, durchgeführt werden. Die Öffnung ist bevorzugt neben der Austragsöffnung angeordnet und kann mit einem Verschluss verschlossen sein oder geschlossen werden.

Es kann vorgesehen sein, dass im Kartuschenkopf eine gasdichte verschließbare Öffnung oberhalb der porösen Scheibe angeordnet ist, die eine Fläche von mindestens 20 mm² hat und die mit der umgebenden Atmosphäre verbunden ist.

Es kann erfindungsgemäß vorgesehen sein, dass der Kolben ein Austragskolben ist, mit dem der gemischte Knochenzementteig durch Vortreiben des Austragskolbens in Richtung Kartuschenkopf aus der Kartusche durch die Austragsöffnung oder durch das Austragsrohr oder den hohlen Mischstab auszupressen ist.

Bevorzugt ist der Kartuschenkopf oben angeordnet und die Rückseite der Kartusche unten, wenn die Lager- und Mischvorrichtung bestimmungsgemäß aufgestellt ist. Ferner ist die Aufnahme bevorzugt unterhalb der der Durchführung angeordnet, beziehungsweise auf einer geringeren Höhe als die Durchführung angeordnet. Damit wird erreicht, dass die Monomerflüssigkeit nicht ungewollt, das heißt nicht ohne Betätigung des Kolbens in Richtung der Rückseite der Kartusche in die Kartusche fließen kann.

Bevorzugt kann auch vorgesehen sein, dass eine Einmündung zum Eintragen der Monomerflüssigkeit in die Aufnahme im Bereich der Ausdrückvorrichtung angeordnet ist, wobei die Einmündung durch das Eindrücken der Ausdrückvorrichtung zu Beginn der Bewegung der Ausdrückvorrichtung in die Aufnahme hinein verschlossen wird.

Hiermit wird sichergestellt, dass die Ausdrückvorrichtung die in der Aufnahme enthaltene Monomerflüssigkeit nicht durch die Einmündung drückt, sondern in die Fluidleitung.

Gemäß einer ersten bevorzugten Ausführungsform kann erfindungsgemäß vorgesehen sein, dass die Aufnahme an der Rückseite der Kartusche angeordnet ist und der Kolben während der Bewegung in Richtung der Rückseite der Kartusche die Ausdrückvorrichtung in die Aufnahme drückt, wobei vorzugsweise der Bereich zwischen der Ausdrückvorrichtung und dem Kolben nach außen geöffnet ist.

Hierdurch kann die Ausdrückvorrichtung mechanisch vom Kolben angetrieben werden, wodurch eine besonders hohe Kraft auf die Ausdrückvorrichtung wirken kann und damit die Monomerflüssigkeit mit einem großen Druck aus der Aufnahme durch die Fluidleitung in den Innenraum der Kartusche gedrückt werden kann. Durch die Öffnung des Bereichs zwischen dem Kolben und der Ausdrückvorrichtung wird sichergestellt, dass eingeschlossene Luft entweichen kann und der Bereich dadurch nicht als Gasfeder wirkt, der den Kolben ungewünscht wieder in Richtung des Kartuschenkopfs bewegt, wenn der Druck auf die Ausdrückvorrichtung nachlässt oder wegfällt.

Bei dieser Ausführungsform kann auch vorgesehen sein, dass der Kolben von der Ausdrückvorrichtung beabstandet ist, so dass bei einer Bewegung des Kolbens in Richtung der Rückseite der Kartusche in einem ersten Abschnitt zunächst ein Unterdruck im Innenraum der Kartusche aufgebaut wird, ohne dass die Ausdrückvorrichtung bewegt wird, und in einem zweiten Abschnitt zusätzlich die Ausdrückvorrichtung in die Aufnahme gedrückt wird.

Hiermit wird sichergestellt, dass zunächst das Gas im Innenraum der Kartusche verdünnt wird und zwischen den Pulver-Partikeln der ersten Ausgangskomponente entweichen kann, bevor die Monomerflüssigkeit in den Innenraum der Kartusche einfließt, beziehungsweise in den Innenraum der Kartusche hinein gedrückt wird.

Ferner kann vorgesehen sein, dass die Ausdrückvorrichtung ein Pumpkolben ist und die Aufnahme ein Hohlzylinder ist, in dem der Pumpkolben axial beweglich ist, wobei auf der Oberseite des Pumpkolbens eine Verlängerung, insbesondere in Form eines zylinderförmigen Körpers, angeordnet ist, die einen Außendurchmesser kleiner oder gleich dem Innendurchmesser des Innenraums der Kartusche hat, wobei der Pumpkolben mit der Verlängerung derart in dem Hohlzylinder angeordnet ist, dass die Verlängerung in den Innenraum der Kartusche ragt.

Hierdurch kann der Pumpkolben auf einfache Weise vom Kolben angetrieben werden, indem der Kolben während seiner Bewegung im Innenraum der Kartusche auf die Verlängerung des Pumpkolbens drückt und diesen dabei in dem Hohlzylinder vortreibt. Dadurch wird die Monomerflüssigkeit aus dem Hohlzylinder heraus und durch die Fluidleitung in die Kartusche hinein gedrückt. Der Kolben selbst kann später als Austragskolben zum Auspressen des gemischten Knochenzementteigs verwendet werden, da sich am Kolben kein Aufsatz befindet, sondern der Aufsatz an dem Pumpkolben angebaut ist.

Gemäß einer zweiten bevorzugten alternativen Ausführungsform kann erfindungsgemäß vorgesehen sein, dass die Aufnahme durch einen zur Kartusche separaten geschlossenen Hohlzylinder begrenzt ist, in dem ein Pumpkolben als Ausdrückvorrichtung axial beweglich gelagert ist, wobei der Pumpkolben umlaufend und dicht mit der Innenwand des Hohlzylinders schließt, wobei der Hohlzylinder an einer ersten, vorzugsweise unteren, Grundfläche mit der Fluidleitung verbunden ist und an einer zweiten, vorzugsweise oberen, Grundfläche über eine Druckgasleitung oder eine Hydraulikleitung an der Rückseite der Kartusche mit dem Innenraum der Kartusche verbunden ist, wobei die Rückseite der Kartusche bis auf die Einmündung in die Druckgasleitung oder die Hydraulikleitung verschlossen ist, so dass bei einer Bewegung des Kolbens in Richtung der Rückseite des Innenraums in dem Raum zwischen dem Kolben und der Rückseite ein Überdruck beziehungsweise ein Druck entsteht, der durch die Druckgasleitung oder die Hydraulikleitung in den Hohlzylinder geleitet wird.

Hierdurch kann der Pumpkolben im Hohlzylinder mit Hilfe des durch die Bewegung des Kolbens entstehenden Überdrucks beziehungsweise Drucks auf eine Hydraulikflüssigkeit in dem Raum zwischen dem Kolben und der Rückseite der Kartusche angetrieben werden. Hierzu liegt der Pumpkolben bevorzugt an der ersten (oberen) Grundfläche des Hohlzylinders an und/oder ist von der zweiten (unteren) Grundfläche des Hohlzylinders beabstandet. Bevorzugt ist der Hohlzylinder fest mit einem Fußteil verbunden.

Die der vorliegenden Erfindung zugrundeliegenden Aufgaben werden auch gelöst durch ein Verfahren zur Vermischung der Ausgangskomponenten eines Knochenzements, insbesondere eines Zweikomponenten-Polymethylmethacrylat-Knochenzements, mit einer erfindungsgemäßen Lager- und Mischvorrichtung, umfassend die folgenden Schritte:
a) der Kolben wird in Richtung der Rückseite des zylindrischen Innenraums der Kartusche bewegt, wobei durch die Bewegung des Kolbens im Innenraum der Kartusche zwischen dem Kolben und dem Kartuschenkopf ein Unterdruck entsteht,
b) durch die Bewegung des Kolbens wird die Ausdrückvorrichtung in die Aufnahme gedrückt, wodurch eine in der Aufnahme enthaltene Monomerflüssigkeit durch die Bewegung der Ausdrückvorrichtung durch die Fluidleitung in den Innenraum der Kartusche gedrückt wird,
c) die Monomerflüssigkeit und die erste Ausgangskomponente werden im Innenraum der Kartusche zu dem Knochenzementteig gemischt, und
d) der Knochenzementteig wird durch Vortreiben des Kolbens in Richtung des Kartuschenkopfs aus dem Innenraum der Kartusche ausgetrieben.

Die Schritte a) und b) können zumindest zeitweise gleichzeitig ablaufen, während die Schritte c) und d) chronologisch nacheinander und auch chronologisch nach den Schritten a) und b) erfolgen. Die Schritte a) und b) können zumindest zeitweise gleichzeitig ablaufen, da zeitweise oder während der gesamten Bewegung des Kolbens die Ausdrückvorrichtung in der Aufnahme angetrieben wird.

Dabei kann vorgesehen sein, dass die Monomerflüssigkeit mit dem Unterdruck im Innenraum der Kartusche aus der Fluidleitung in den Innenraum der Kartusche gesaugt wird.

Hierdurch wird erreicht, dass neben dem Druck der Ausdrückvorrichtung auch der in der Kartusche entstehende Unterdruck zur Förderung der Monomerflüssigkeit aus der Aufnahme verwendet wird. Der Unterdruck im Innenraum der Kartusche wird schon während der Bewegung des Kolbens aufgebaut und dieser Unterdruck kann bereits ein Einsaugen der Monomerflüssigkeit in den Innenraum der Kartusche bewirken.

Ferner kann vorgesehen sein, dass vor Schritt a) die Monomerflüssigkeit in die Aufnahme eingeleitet wird, insbesondere nach Öffnen eines Monomerflüssigkeitsbehälters in einem Behälter der Lager- und Mischvorrichtung.

Hiermit wird das Verfahren für Full-Prepacked-Systeme anwendbar, da die Monomerflüssigkeit in einem dafür vorgesehenen Monomerflüssigkeitsbehälter, wie beispielsweise einer Glasampulle oder einem metallbeschichteten Folienbeutel auch längerfristig in der Lager- und Mischvorrichtung gelagert werden kann.

Des Weiteren kann vorgesehen sein, dass in Schritt a) der Kolben mit einem Mischstab in Richtung der Rückseite der Kartusche gedrückt wird, wobei der Mischstab beweglich in einer gasdichten Durchführung, insbesondere in der Austragsöffnung, im Kartuschenkopf gelagert ist, und in Schritt c) die Mischung der Monomerflüssigkeit mit der ersten Ausgangskomponente durch Bewegen einer mit dem Mischstab verbundenen Mischeinrichtung im Innenraum der Kartusche zwischen dem Kolben und dem Kartuschenkopf erfolgt, indem der Mischstab und damit die Mischeinrichtung bewegt wird.

Hierdurch wird der Mischstab, der zum Bewegen der Mischeinrichtung in dem Innenraum der Kartusche vorhanden ist, gleichzeitig zum Bewegen des Kolbens und damit zum Bewegen der Ausdrückvorrichtung und zum Erzeugen des Unterdrucks im Innenraum der Kartusche verwendet. Besonders bevorzugt kann dieser Mischstab als Rohr ausgeführt sein und so zusätzlich auch noch als Austragsrohr zum Applizieren des gemischten Knochenzementteigs verwendet werden.

Dabei kann wiederum vorgesehen sein, dass zwischen Schritt c) und d) die Mischeinrichtung mit dem Mischstab zum Kartuschenkopf gezogen wird und ein Kern aus dem Mischstab entfernt wird, so dass der Mischstab ohne den Kern ein Austragsrohr bildet, durch das der gemischte Knochenzementteig in Schritt d) aus dem Innenraum der Kartusche ausgepresst wird.

Hiermit wird der Mischstab zusätzlich auch noch als Austragsrohr nutzbar und die Austragsöffnung kann zur Durchführung des Mischstabs genutzt werden.

Mit einer Weiterentwicklung des erfindungsgemäßen Verfahrens kann vorgesehen sein, dass vor Schritt d) die Kartusche von der Lager- und Mischvorrichtung getrennt wird und in eine Auspressvorrichtung eingesetzt wird, mit der in Schritt d) der Kolben mittels eines Stößels oder einer vortreibbaren Stange in Richtung des Kartuschenkopfs gedrückt wird, um den Knochenzementteig aus dem Innenraum der Kartusche auszutreiben.

Hiermit kann die Kartusche getrennt von der Lager- und Mischvorrichtung zum Applizieren des bereits fertig gemischten Knochenzementteigs verwendet werden. Dadurch wird die Handhabung während der Applikation vereinfacht.

Des Weiteren kann vorgesehen sein, dass die Aufnahme ein Hohlzylinder ist und die Ausdrückvorrichtung ein Pumpkolben ist, der in Längsrichtung in dem Hohlzylinder beweglich angeordnet ist, wobei der Pumpkolben durch Aufdrücken des Kolbens im Hohlzylinder bewegt wird oder durch einen zwischen der Rückseite der Kartusche und dem Kolben im Innenraum erzeugten Überdrück oder Druck pneumatisch oder hydraulisch bewegt wird, wobei vorzugsweise der Überdruck durch eine Druckgasleitung in den Hohlzylinder geleitet wird oder der Druck durch eine Hydraulikleitung in den Hohlzylinder geleitet wird.

Hierdurch wird die Kraft, die zum Bewegen des Pumpkolbens benötigt wird, auf einfache Weise aus der Bewegung des Kolbens in der Kartusche entnommen. Hiermit muss lediglich der Kolben angetrieben werden, um eine Vielzahl verschiedener Vorgänge in der Lager- und Mischvorrichtung durchzuführen.

Es kann auch vorgesehen sein, dass vor den Schritten a) und b) ein Monomerflüssigkeitsbehälter, insbesondere die Glasampulle oder der Folienbeutel, in der Lager- und Mischvorrichtung geöffnet wird, wobei die Monomerflüssigkeit aus dem Monomerflüssigkeitsbehälter ausläuft und in die Aufnahme fließt, vorzugsweise durch ein Sieb und/oder einen Filter in die Aufnahme fließt.

Es kann erfindungsgemäß auch vorgesehen sein, dass vor Schritt a) der Kartuschenkopf gasdicht verschlossen wird. Hiermit wird sichergestellt, dass in dem Innenraum der Kartusche ein Unterdruck erzeugt werden kann, unter dem die Ausgangskomponenten gemischt werden können und der gegebenenfalls auch zum Einsaugen der Monomerflüssigkeit verwendet werden kann. Gleichzeitig kann aber zuvor durch den geöffneten Kartuschenkopf das Innere der Kartusche mit Hilfe eines sterilisierenden Gases, wie beispielsweise Ethylenoxid, sterilisiert werden.

Erfindungsgemäße Lager- und Mischvorrichtungen und erfindungsgemäße Verfahren sind theoretisch auch mit einer Mehrzahl von Kartuschen umsetzbar, in denen eine jeweils ein beweglicher Kolben angeordnet ist. Derartige Lösungen werden als im Rahmen der vorliegenden Erfindung liegend verstanden, da dort ja auch immer jeweils eine Kartusche erfindungsgemäß betrieben wird, beziehungsweise betrieben werden kann.

Der Erfindung liegt die überraschende Erkenntnis zugrunde, dass der Kolben, der auch zum Austragen des gemischten Knochenzementteigs aus der Kartusche verwendet wird, zuvor zum Antreiben einer Ausdrückvorrichtung verwendet werden kann, mit der eine Monomerflüssigkeit aus einer Aufnahme in den Innenraum der Kartusche gedrückt werden kann, um diese dort mit dem Knochenzement zu mischen. Ferner wurde überraschend gefunden, dass der gleiche Kolben zum Erzeugen eines Unterdrucks und damit zum Einsaugen der Monomerflüssigkeit verwendet werden kann. Wenn der Mischstab zudem noch zur Bedienung beziehungsweise zur Bewegung des Kolbens zum Erzeugen des Unterdrucks verwendet wird, können hierbei zusätzliche Bauteile vermieden werden. Ferner kann der Mischstab auch als Austragsrohr verwendet werden, um die Anzahl der notwendigen Bauteile zu minimieren. Die Idee besteht dabei darin, dass der Kolben nicht bereits an der Rückseite des Innenraums der Kartusche anliegt, sondern von diesem beabstandet angeordnet ist. Der Hub des Kolbens bis zur Rückseite des Innenraums der Kartusche kann dann genutzt werden, um die Ausdrückvorrichtung anzutreiben, mit dem die Monomerflüssigkeit in den Innenraum der Kartusche gedrückt werden kann, und im Innenraum der Kartusche einen Unterdruck zu erzeugen, der zum Mischen eines blasenfreien oder blasenarmen Knochenzements genutzt werden kann und mit dem die Monomerflüssigkeit in den Innenraum der Kartusche gesaugt werden kann. Der verbleibende Unterdruck kann also dazu genutzt werden, die Ausgangskomponenten unter Unterdruck miteinander zu mischen, um im entstehenden Knochenzementteig möglichst wenige Gas- beziehungsweise Lufteinschlüsse entstehen zu lassen.

Eine erste erfindungsgemäße Lager- und Mischvorrichtung für Polymethylmethacrylat-Knochenzement ist beispielsweise zusammengesetzt aus
a) einer zylinderförmigen Kartusche,
b) einem in der zylinderförmigen Kartusche axial beweglichen Kolben,
c) einem Kartuschenkopf mit einer Durchführung für einen Mischstab,
d) einem Mischstab, der durch die Durchführung des Kartuschenkopfs axial verschiebbar ist,
e) einer Mischeinrichtung, die mit dem im Innenraum der Kartusche angeordneten Ende des Mischstabs verbunden ist,
f) einem Betätigungsmittel, das am äußeren Ende des Mischstabs angeordnet ist,
g) mindestens einem außerhalb der Kartusche angeordneten Monomerflüssigkeitsbehälter,
h) einer Öffnungsvorrichtung für den Monomerflüssigkeitsbehälter,
i) wobei der mindestens eine Monomerflüssigkeitsbehälter mit einem Hohlzylinder flüssigkeitsdurchlässig durch eine Öffnung verbunden ist, wobei der Monomerflüssigkeitsbehälter oberhalb der Öffnung angeordnet ist
j) einer Fluidleitung, die den Hohlzylinder an einem verschlossenen Boden des Hohlzylinders mit dem von der Kartusche, dem Kartuschenkopf und dem Kolben gebildeten Hohlraum flüssigkeitsdurchlässig verbindet,
k) einem Zementpulver, das in dem von der Kartusche, dem Kartuschenkopf und dem Kolben gebildeten Hohlraum angeordnet ist,
l) wobei der Hohlzylinder an der Oberseite mit einem Deckel verschlossen ist, der mit einer Druckgasleitung mit einer Kartuschenaufnahme gasdurchlässig verbunden ist,
m) wobei oberhalb der Öffnung im Hohlzylinder ein axial beweglicher Pumpkolben im Hohlzylinder angeordnet ist und wobei die Kartusche lösbar und gasdicht mit der Kartuschenaufnahme verbunden ist.

Eine weitere alternative erfindungsgemäße Lager- und Mischvorrichtung für Polymethylmethacrylat-Knochenzement ist beispielsweise zusammengesetzt aus
a) einer zylinderförmigen Kartusche,
b) einem in der zylinderförmigen Kartusche axial beweglichen Kolben,
c) einem Kartuschenkopf mit einer Durchführung für einen Mischstab,
d) einem Mischstab, der durch die Durchführung des Kartuschenkopfs axial verschiebbar ist,
e) einer Mischeinrichtung, die mit dem im Innenraum der Kartusche angeordneten Ende des Mischstabs verbunden ist,
f) einem Betätigungsmittel, das am äußeren Ende des Mischstabs angeordnet ist,
g) mindestens einem außerhalb der Kartusche angeordneten Monomerflüssigkeitsbehälter,
h) einer Öffnungsvorrichtung für den Monomerflüssigkeitsbehälter,
i) einem Zementpulver, das in dem von der Kartusche, dem Kartuschenkopf und dem Kolben gebildeten Hohlraum angeordnet ist,
j) wobei unterhalb der Kartusche eine ringförmige Kartuschenaufnahme in einem Fußteil angeordnet ist, wobei die Kartusche lösbar mit der Kartuschenaufnahme verbunden ist,
k) einem Hohlzylinder, der unterhalb der Kartuschenaufnahme angeordnet ist, wobei der mindestens eine Monomerflüssigkeitsbehälter mit dem Hohlzylinder flüssigkeitsdurchlässig durch eine Öffnung verbunden ist und wobei der Monomerflüssigkeitsbehälter oberhalb der Öffnung angeordnet ist,
l) wobei der Hohlzylinder an der Unterseite mit einem Boden verschlossen ist, der flüssigkeitsdurchlässig mit der Fluidleitung verbunden ist,
m) wobei oberhalb der Öffnung im Hohlzylinder ein axial beweglicher Pumpkolben im Hohlzylinder angeordnet ist, und
n) wobei auf der Oberseite des Pumpkolbens ein zylinderförmiger Körper angeordnet ist, der einen Außendurchmesser kleiner oder gleich dem Innendurchmesser der Kartusche hat, wobei der Pumpkolben mit dem zylinderförmigen Körper so in dem Hohlzylinder angeordnet ist, dass das obere Ende des zylinderförmigen Körpers in den Innenraum der Kartusche ragt.

Bevorzugt kann dabei vorgesehen sein, dass der Kolben derart axial in der Kartusche angeordnet ist, dass der von der Kartusche, dem Kartuschenboden und dem Kolben gebildete Hubraum ein Volumen gleich oder größer dem gesamten Volumen der Fluidleitung und des mindestens einen Monomerflüssigkeitsbehälters hat.

Die Durchführung für den Mischstab ist durch die Austragsöffnung realisiert, beziehungsweise wird vorliegend als solche bezeichnet, wenn der Mischstab als Austragsrohr verwendet wird. Das Betätigungsmittel kann beispielsweise ein Griff sein, mit dem der Mischstab manuell bedienbar ist.

Erfindungsgemäß kann dabei ein Fußteil vorgesehen sein, mit dem die Kartusche lösbar verbunden ist und das mit dem mindestens einem Monomerflüssigkeitsbehälter unlösbar verbunden ist.

Es kann ferner vorgesehen sein, dass im Kartuschenkopf eine für Gase und Flüssigkeiten durchlässige poröse Scheibe angeordnet ist, die mit einer gasdurchlässigen Durchführung verbunden ist und die den Hohlraum durchlässig für Gase und Flüssigkeiten und undurchlässig für Feststoffpartikel abschließt.

Im Kartuschenkopf kann eine gasdicht verschließbare Öffnung oberhalb der porösen Scheibe angeordnet sein, die mindestens eine Fläche von mindestens 20 mm² hat und die mit der umgebenden Atmosphäre verbunden ist.

Erfindungsgemäß kann des Weiteren vorgesehen sein, dass der Kolben mindestens ein Rastelement besitzt, das mit einem Gegenrastelement an der Innenseite der Kartusche oberhalb des Kartuschenbodens lösbar gerastet werden kann.

Erfindungsgemäß ist beispielsweise ein Verfahren zum Mischen und Austragen von Polymethylmethacrylat-Knochenzement. Das Verfahren ist durch folgende nacheinander ablaufende Schritte gekennzeichnet,
a) gasdichtes Verschließen des Kartuschenkopfs,
b) Öffnen des mindestens einen Monomerflüssigkeitsbehälters,
c) Fließen der Monomerflüssigkeit aus dem Monomerflüssigkeitsbehälter durch die Öffnung in den Hohlzylinder,
d) Drücken des Betätigungsmittels des Mischstabs in Richtung Kartuschenboden (Rückseite des Innenraums der Kartusche),
e) Verschieben des Kolbens durch den Mischstab in Richtung des Kartuschenbodens,
f) Erzeugen eines Unterdrucks in dem von der Kartusche, dem Kartuschenkopf und dem Kolben gebildeten Hohlraum (im Innenraum der Kartusche),
g) Erzeugen eines Überdrucks in dem von der Kartusche, der geschlossenen Rückseite der Kartusche und dem Kolben gebildeten Hohlraum (im Innenraum der Kartusche),
h) Weiterleitung des Überdrucks durch eine Druckgasleitung in den Hohlzylinder oberhalb des axial verschiebbaren Pumpkolbens,
i) Bewegen des Pumpkolbens in Richtung des Bodens des Hohlzylinders,
j) Transfer der Monomerflüssigkeit von dem Hohlzylinder durch die Fluidleitung in den Innenraum der Kartusche durch den gebildeten Unterdruck und durch Auspressen aus dem Hohlzylinder durch den in Richtung des Bodens bewegten Pumpkolben,
k) Mischen des Zementpulvers mit der Monomerflüssigkeit durch manuelle Betätigung des Betätigungselements des Mischstabs durch axiale und drehende Bewegung der Mischeinrichtung,
l) Ziehen des Mischstabs zum Kartuschenkopf,
m) Entfernen eines Kerns aus dem hohlen Mischstab,
n) Einsetzen der Kartusche in eine Auspressvorrichtung,
o) Betätigung der Auspressvorrichtung, wobei der Kolben in Richtung des Kartuschenkopfs bewegt wird, und
p) Auspressen des gemischten Knochenzementteigs aus der Kartusche durch den hohlen Mischstab.

Das Verfahren ist auf die oben genannte erste erfindungsgemäße Lager- und Mischvorrichtung abgestellt und gibt deren Funktionsweise wieder.

Erfindungsgemäß ist beispielsweise auch ein alternatives Verfahren zum Mischen und Austragen von Polymethylmethacrylat-Knochenzement. Das alternative Verfahren ist durch folgende nacheinander ablaufende Schritte gekennzeichnet,
a) gasdichtes Verschließen des Kartuschenkopfs,
b) Öffnen des mindestens einen Monomerflüssigkeitsbehälters,
c) Fließen der Monomerflüssigkeit aus dem Monomerflüssigkeitsbehälter durch die Öffnung in den Hohlzylinder,
d) Drücken des Betätigungsmittels des Mischstabs in Richtung Kartuschenboden (Rückseite des Innenraums der Kartusche),
e) Verschieben des Kolbens durch den Mischstab in Richtung des Kartuschenbodens,
f) Erzeugen eines Unterdrucks in dem von der Kartusche, dem Kartuschenkopf und dem Kolben gebildeten Hohlraum (im Innenraum der Kartusche),
g) Verschiebung des zylinderförmigen Körpers des Pumpkolbens durch axiale Verschiebung des Kolbens in Richtung der Rückseite der Kartusche,
h) Transfer der Monomerflüssigkeit von dem Hohlzylinder durch die Fluidleitung in den Innenraum der Kartusche durch den gebildeten Unterdruck und durch Auspressen aus dem Hohlzylinder durch den in Richtung des Bodens bewegten Pumpkolben,
i) Mischen des Zementpulvers mit der Monomerflüssigkeit durch manuelle Betätigung des Betätigungselements des Mischstabs durch axiale und drehende Bewegung der Mischeinrichtung,
j) Ziehen des Mischstabs zum Kartuschenkopf,
k) Entfernen eines Kerns aus dem hohlen Mischstab,
l) Einsetzen der Kartusche in eine Auspressvorrichtung,
m) Betätigung der Auspressvorrichtung, wobei der Kolben in Richtung des Kartuschenkopfs bewegt wird, und
n) Auspressen des gemischten Knochenzementteigs aus der Kartusche durch den hohlen Mischstab.

Das alternative Verfahren ist auf die oben genannte weitere, alternative erfindungsgemäße Lager- und Mischvorrichtung abgestellt und gibt deren Funktionsweise wieder.

Im Folgenden werden weitere Ausführungsbeispiele der Erfindung anhand von dreizehn schematisch dargestellten Figuren erläutert, ohne jedoch dabei die Erfindung zu beschränken. Dabei zeigt:
Figur 1: eine schematische Querschnittansicht einer ersten beispielhaften erfindungsgemäßen Lager- und Mischvorrichtung im Ausgangszustand;
Figur 2: eine schematische perspektivische Ansicht der Lager- und Mischvorrichtung nach Figur 1;
Figur 3: eine schematische Querschnittansicht der ersten beispielhaften Lager- und Mischvorrichtung mit geöffnetem Monomerflüssigkeitsbehälter;
Figur 4: eine schematische Querschnittansicht der ersten beispielhaften Lager- und Mischvorrichtung während beziehungsweise nach dem Eindrücken der Monomerflüssigkeit;
Figur 5: eine schematische Teilquerschnittansicht der ersten beispielhaften Lager- und Mischvorrichtung mit gemischtem Knochenzementteig;
Figur 6: eine schematische Querschnittansicht des unteren Teils der ersten beispielhaften Lager- und Mischvorrichtung mit einer Schnittebene senkrecht zu den der Figuren 1, 3, 4 und 5;
Figur 7: eine schematische Querschnittansicht einer zweiten beispielhaften erfindungsgemäßen Lager- und Mischvorrichtung im Ausgangszustand;
Figur 8: eine schematische perspektivische Ansicht der zweiten beispielhaften Lager- und Mischvorrichtung nach Figur 7;
Figur 9: eine schematische Querschnittansicht der zweiten beispielhaften Lager- und Mischvorrichtung mit geöffnetem Monomerflüssigkeitsbehälter;
Figur 10: eine schematische Querschnittansicht der zweiten beispielhaften Lager- und Mischvorrichtung während dem Eindrücken der Monomerflüssigkeit;
Figur 11: eine schematische Querschnittansicht der zweiten beispielhaften Lager- und Mischvorrichtung nach dem Eindrücken der Monomerflüssigkeit;
Figur 12: eine schematische Teilquerschnittansicht der zweiten beispielhaften Lager- und Mischvorrichtung mit gemischtem Knochenzementteig; und
Figur 13: zwei Ausschnittvergrößerungen des oberen Bereichs der zweiten erfindungsgemäßen Lager- und Mischvorrichtung, links eine schematische perspektivische Ansicht und rechts eine schematische Teilquerschnittansicht.

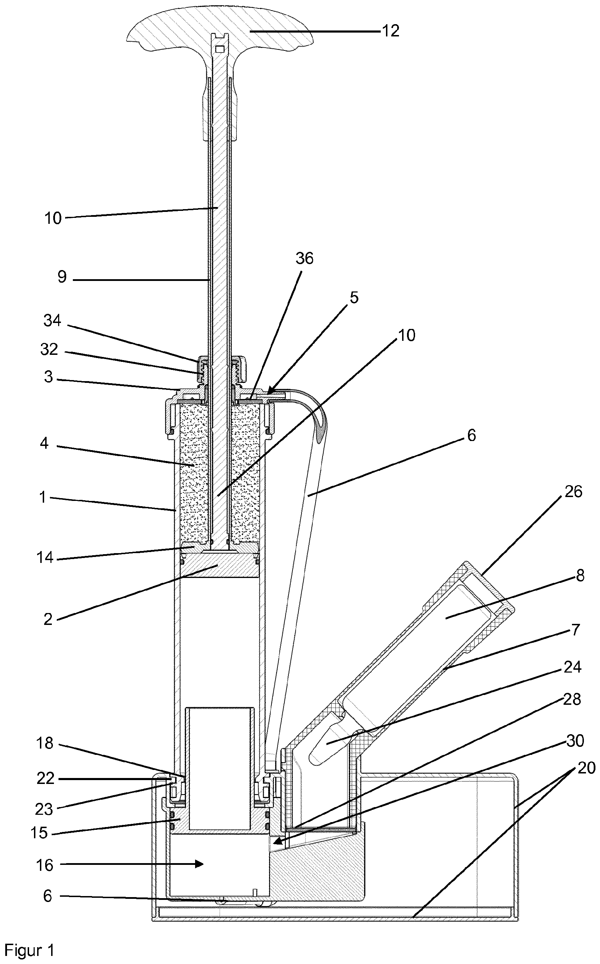

Die Figuren 1 bis 6 zeigen eine beispielhafte Ausführung einer ersten erfindungsgemäßen Lager- und Mischvorrichtung. Figur 1 zeigt dabei eine schematische Querschnittansicht der ersten beispielhaften erfindungsgemäßen Lager- und Mischvorrichtung im Ausgangszustand. Die Lager- und Mischvorrichtung umfasst eine Kartusche 1 mit einem zylindrischen Innenraum, in dem ein Kolben 2 linear axial beweglich angeordnet ist. An der Vorderseite der Kartusche 1 (Figuren 1 und 3 bis 5 oben) ist der Innenraum beziehungsweise die Kartusche 1 durch einen Kartuschenkopf 3 nach Art eines Deckels oder einer Kappe verschlossen. Der Kartuschenkopf 3 ist gegen die Kartusche 1 mit einer umlaufenden Dichtung abgedichtet. In dem Innenraum der Kartusche 1 befindet sich zwischen dem Kolben 2 und dem Kartuschenkopf 3 ein Knochenzementpulver 4 als erste Ausgangskomponente 4 eines PMMA-Knochenzements.

Der Kolben 2 schließt mit einer umlaufenden Gummidichtung gasdicht mit der Innenwand des Innenraums der Kartusche 1 ab. Dadurch kann durch eine Bewegung des Kolbens 2 vom Kartuschenkopf 3 weg im Innenraum der Kartusche 1 ein Unterdruck entstehen, der durch eine Durchführung 5 im Kartuschenkopf 3 zu einer Fluidleitung 6 geleitet wird, beziehungsweise auf die Fluidleitung 6 einwirkt.

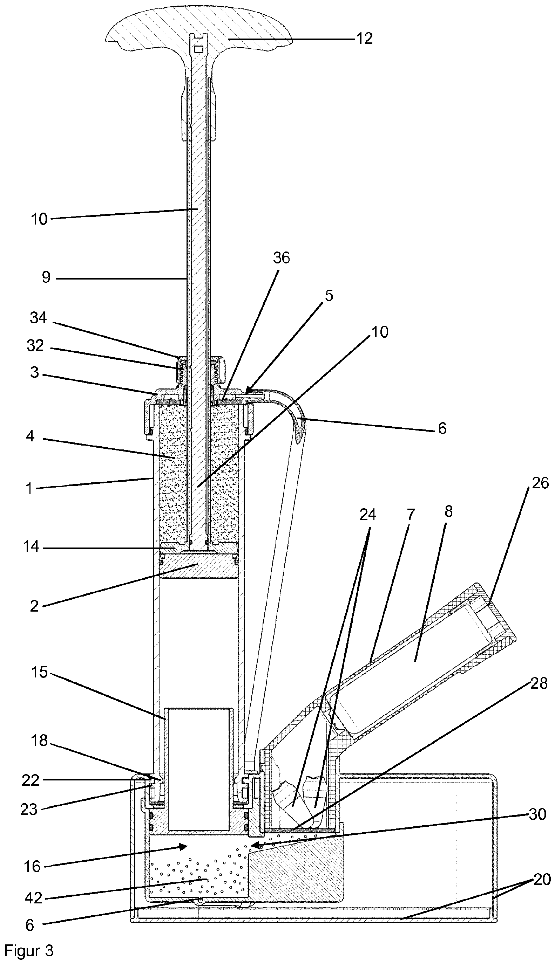

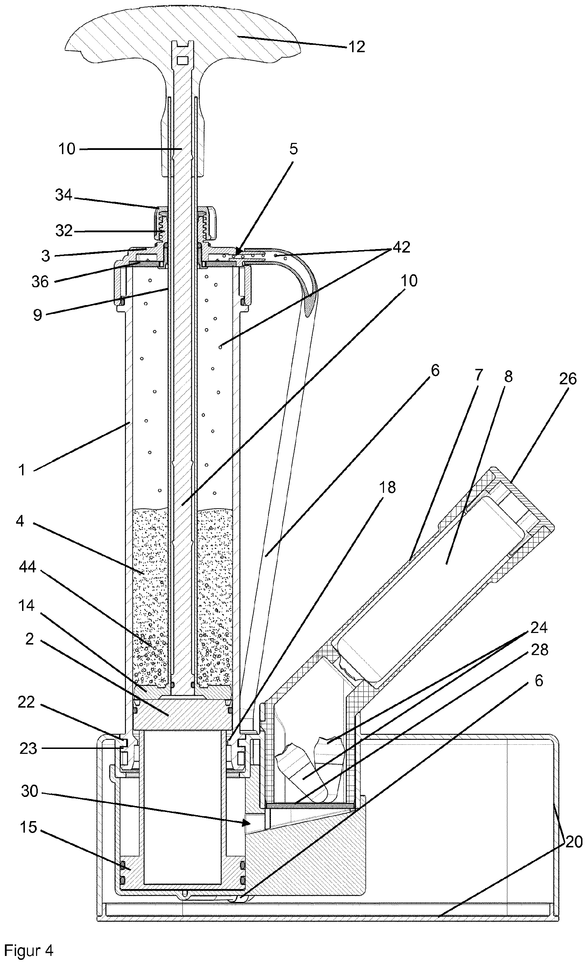

Ein zur Kartusche 1 separater Behälter 7 ist neben der Kartusche 1 angeordnet. In dem Behälter 7 ist eine Glasampulle 8 kopfüber eingesteckt ist. Die Glasampulle 8 ist in den geschnittenen Ansichten der Figuren 1 und 3 bis 5 nicht geschnitten dargestellt und ist in Figur 1 mit einer Monomerflüssigkeit 42 (siehe Figuren 3 und 4) gefüllt.

In dem Kartuschenkopf 3 befindet sich eine zentrale Austragsöffnung, durch die ein hohler beziehungsweise rohrförmiger Mischstab 9 hindurchgeführt ist. Der hohle Mischstab 9 ist in seinem Inneren mit einem Kern 10 verschlossen, der sich aus dem Mischstab 9 herausziehen lässt. Der Mischstab 9 ist mit einer umlaufenden Dichtung im Bereich der Austragsöffnung gegen den Kartuschen kopf 3 abgedichtet, so dass der Unterdruck im Innenraum der Kartusche 1 nicht (oder zumindest nicht schnell) Luft zwischen dem Kartuschenkopf 3 und dem Mischstab 9 in den Innenraum der Kartusche 1 nachziehen kann, auch wenn der Mischstab 9 in der Austragsöffnung gedreht und in Längsrichtung bewegt wird.

An dem vorderen Ende des Mischstabs 9 ist ein Griff 12 angeordnet, mit dem der Mischstab 9 manuell gegen die Kartusche 1 in Längsrichtung bewegt und axial gedreht werden kann. Zudem kann mit dem Griff 12 nach Lösen einer Rastung (nicht gezeigt) oder nach Überwinden eines Widerstands der Kern 10 aus dem Mischstab 9 herausgezogen werden.

Am Mischstab 9 ist im Innenraum der Kartusche 1 zwischen dem Kolben 2 und dem Kartuschenkopf 3 eine Mischeinrichtung 14 mit mehreren Mischflügeln 14 befestigt, so dass sich beim Drehen und Bewegen des Mischstabs 9 gegen die Kartusche 1 auch die Mischeinrichtung 14 im Innenraum der Kartusche 1 dreht und axial bewegt. Damit kann über den Mischstab 9 der Inhalt des Innenraums der Kartusche 1 mit Hilfe der Mischeinrichtung 14 mechanisch durchmischt werden.

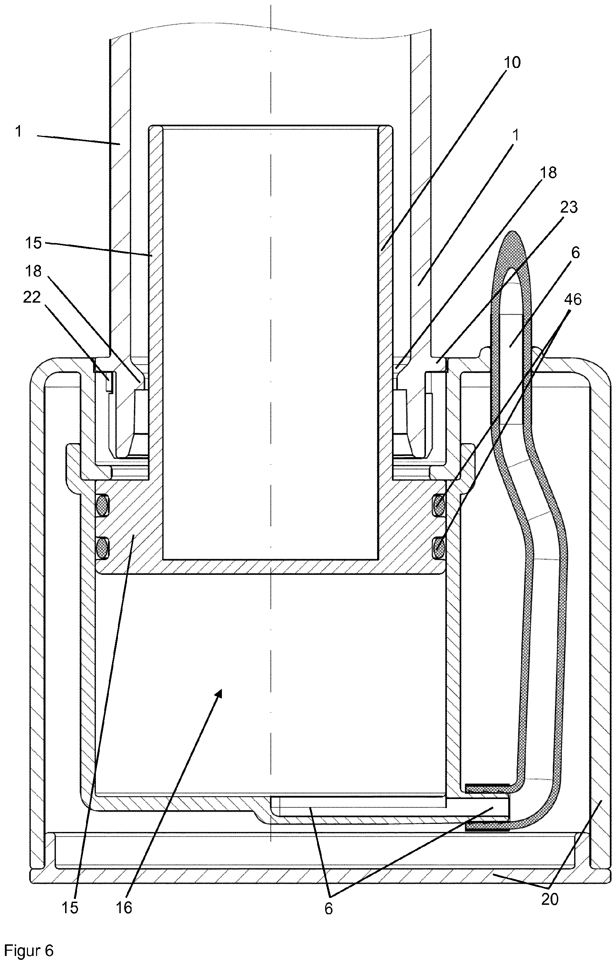

In die Rückseite der Kartusche 1 ist ein Pumpkolben 15 eingesteckt, der an seiner Vorderseite einen zylindrischen Aufsatz als Verlängerung aufweist, der in den Innenraum der Kartusche 1 unterhalb des Kolbens 2 hineinragt, aber in seiner Ausgangsstellung (siehe Figur 1) vom Kolben 2 beabstandet ist. Der Pumpkolben 15 ist in einer zylindrischen Aufnahme 16 in Längsrichtung beweglich gelagert und gegen die Innenwand der zylindrischen Aufnahme 16 mit zwei umlaufenden Dichtungen 46 (siehe Figur 6) abgedichtet. Die Aufnahme 16 ist dazu angrenzend und unterhalb der Kartusche 1 angeordnet. An der Unterseite der Aufnahme 16 mündet die Fluidleitung 6 in die Aufnahme 16.

Die Fluidleitung 6 erstreckt sich bereichsweise parallel zur Achse der zylindrischen Kartusche 1 nach unten vom Kartuschenkopf 3 weg, vollführt dann eine Kurve und erstreckt sich dann bis zur Einmündung in die Aufnahme 16 nach oben. Die Aufnahme 16 könnte auch als Verbreiterung der Fluidleitung 6 aufgefasst werden. Die Aufnahme 16 bildet ein Reservoir für die Monomerflüssigkeit 42 aus der Glasampulle 8.

Um im Innenraum der Kartusche 1 einen Unterdruck zu erzeugen, kann der Kolben 2 in Richtung einer Rückseite der Kartusche 1 (bodenseitig, das heißt in den Figuren 1 bis 6 nach unten) beziehungsweise in Richtung der Rückseite des Innenraums der Kartusche 1 bewegt werden, indem der Kolben 2 mit Hilfe des Mischstabs 9 manuell in Richtung der Rückseite gedrückt wird. Damit der Kolben 2 nicht bodenseitig aus dem Innenraum der Kartusche 1 herausgedrückt werden kann, ist an der Innenseite der Kartusche 1 im Bereich der Rückseite des Innenraums ein Anschlag 18 in Form einer umlaufenden Erhebung vorgesehen. Der Kolben 2 kann also nur bis zum Anschlag 18 in Richtung der Rückseite der Kartusche 1 gedrückt werden. Zuvor blockiert allerdings schon der Pumpkolben 15 die Bewegung, wenn er am Boden der Aufnahme 16 angelangt ist. Nach dem Entfernen der Kartusche 1 aus der Lager- und Mischvorrichtung wird mit dem Anschlag 18 jedoch sichergestellt, dass der Kolben 2 nicht aus der Rückseite der Kartusche 1 austreten kann.

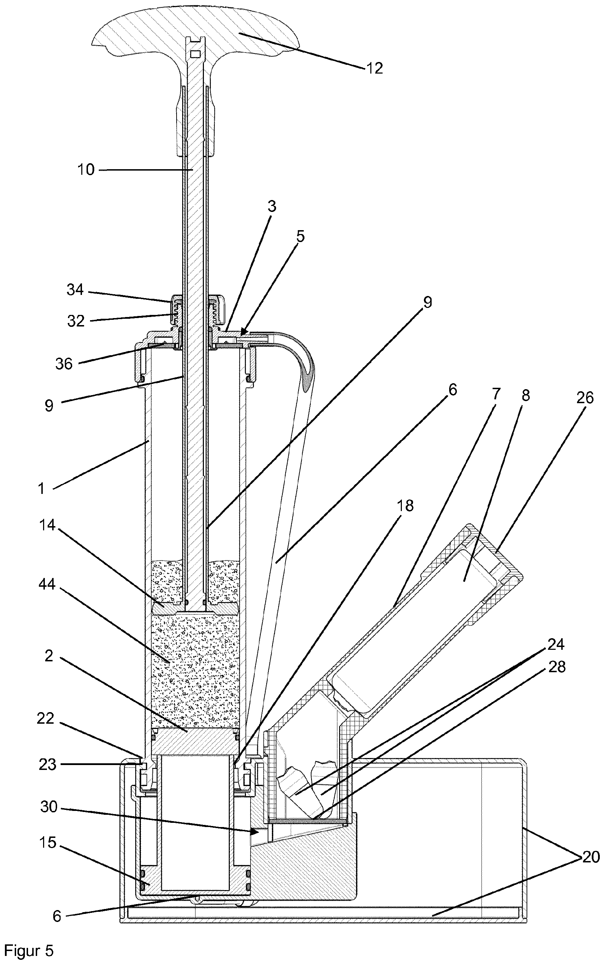

Die Kartusche 1 ist nämlich lösbar mit einem Fußteil 20 verbunden. An der Rückseite der Kartusche 1 sind zur Befestigung mit dem Fußteil 20 außen zwei umlaufende Vorsprünge 22 angeordnet. Hierzu wird die Kartusche 1 bodenseitig, also im Bereich der Rückseite mit einer Halterung 23 mit dem Fußteil 20 gerastet. Diese Vorsprünge 22 können zudem zur Befestigung eine Auspressvorrichtung (nicht gezeigt) verwendet werden, mit der der fertig gemischte Knochenzementteig 44 (siehe Figur 5) appliziert werden kann. Dazu wird der Kolben 2 als Austragskolben 2 mit der Auspressvorrichtung durch eine vortreibbare Stange in die Kartusche 1 in Richtung des Kartuschenkopfs 3 eingepresst und dabei der Knochenzementteig 44 durch das Austragsrohr 9 beziehungsweise den hohlen Mischstab 9 ausgepresst.

Die Glasampulle 8 weist einen abbrechbaren Ampullenkopf 24 auf. Wenn der Ampullenkopf 24 abgebrochen oder aufgebrochen wird, wird damit die Glasampulle 8 geöffnet. Der Behälter 7 ist aus einem elastisch verformbaren Material gefertigt, das eine Verdickung am Hals zwischen dem Ampullenkopf 24 und dem Körper der Ampulle 8 aufweist. Dadurch ist der Ampullenkopf 24 fest gelagert, während der Körper der Ampulle 8 aufgrund der Elastizität des den Behälter 7 bildenden Einsatzes bewegt werden kann. So kann die Glasampulle 8 innerhalb der Lager- und Mischvorrichtung aufgebrochen werden. An der Rückseite des Behälters 7 ist ein Stopfen 26 als Deckel 26 vorgesehen, mit dem die Glasampulle 8 in Position gehalten wird. In dem Deckel 26 sind zwei Öffnungen vorgesehen, durch die Luft ins Innere des Behälters 7 nachströmen kann.

In der Verbindung zwischen dem Behälter 7 und der Aufnahme 16 beziehungsweise unterhalb des Behälters 7 ist ein Filter 28 und/oder Sieb 28 angeordnet, mit dem Bruchstücke beziehungsweise Glassplitter der Glasampulle 8, die beim Aufbrechen der Glasampulle 8 entstehen können, zurückgehalten werden. Unterhalb der Filters 28 und/oder Siebs 28 neigt sich der Boden und der Behälter 7 und die Aufnahme 16 sind über eine Öffnung 30 flüssigkeitsdurchlässig miteinander verbunden. Nach dem Öffnen der Glasampulle 8 fließt die Monomerflüssigkeit 42 nach unten ab über eine geneigte Fläche und durch die Öffnung 30 in dir Aufnahme 16 hinein (siehe Figur 3). Der abgebrochene Ampullenkopf 24 und eventuell entstehende Glassplitter werden dabei von dem Sieb 28 und/oder dem Filter 28 zurückgehalten.

Im Bereich der Austragsöffnung ist am Kartuschenkopf 3 ein Stutzen 32 mit einem Außengewinde vorgesehen. Auf dieses Außengewinde kann eine konische Überwurfmutter 34 als Befestigungseinrichtung 34 aufgeschraubt werden, um den Mischstab 9 gegen den Kartuschenkopf 3 zu fixieren. Um den Mischstab 9 gegen den Kartuschenkopf 3 fixieren zu können, wird die Überwurfmutter 34 weiter auf das Außengewinde des Stutzens 32 geschraubt. Um dies zunächst zu verhindern, beziehungsweise um eine ungewollte Fixierung zu vermeiden, kann eine herausziehbare Sicherung (nicht gezeigt) in Form eine Spange mit einer Greiflasche vorgesehen, die zwischen der Überwurfmutter 34 und dem Kartuschenkopf 3 angeordnet ist. Wenn die Sicherung abgezogen wird, kann die Überwurfmutter 34 weiter auf das Außengewinde des Stutzens 32 geschraubt werden, so dass der Stutzen 32 zusammengedrückt wird und dadurch den Mischstab 9 gegen den Kartuschenkopf 3 fixiert.

Eine zusätzliche seitliche Öffnung ist im Kartuschenkopf 3 vorgesehen, durch die der Innenraum der Kartusche 1 für sterilisierende Gase, wie Ethylenoxid, zugänglich ist. Vorliegend ist die seitliche Öffnung mit einem Verschluss 40 verschlossen.

Zwischen dem Kartuschenkopf 3 und dem Innenraum der Kartusche 1 ist eine Porenscheibe 36 beziehungsweise ein Porenfilter 36 angeordnet, der für Gase und Flüssigkeiten durchlässig ist aber für Pulver und Feststoffe wie das Knochenzementpulver 4 undurchlässig ist, so dass die Durchführung 5 nur über den Porenfilter 36 mit dem restlichen Innenraum der Kartusche 1 verbunden ist. Der Porenfilter 36 verhindert ein Vordringen des Knochenzementpulvers 4 in die Fluidleitung 6 und damit eine ungewollte vorzeitige Reaktion des Knochenzementpulvers 4 mit der Monomerflüssigkeit 42 und damit einen ungewollten Verschluss des dünnen Teils der Fluidleitung 6.

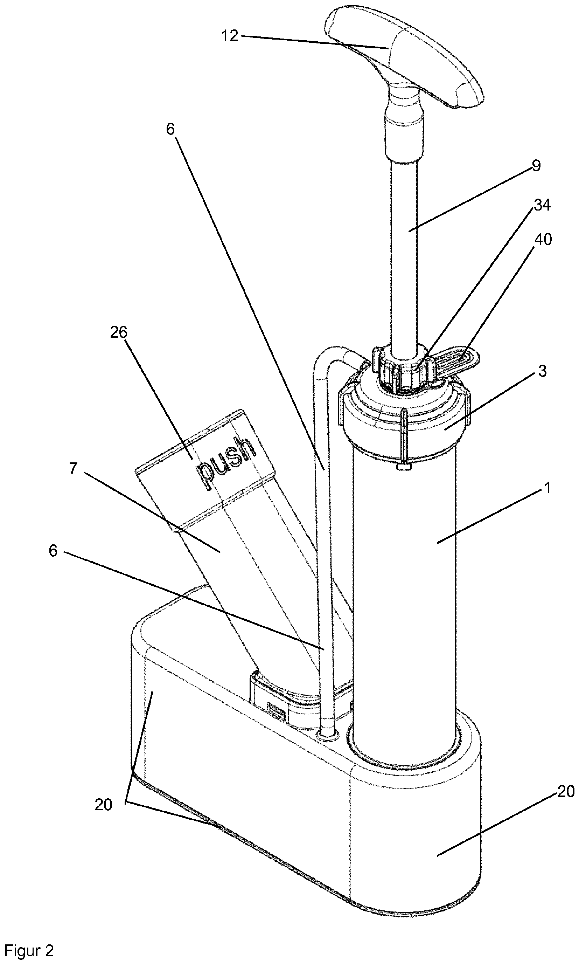

Anhand der Figuren 1 bis 6 wird im Folgenden ein exemplarisches erfindungsgemäßes Verfahren vorgestellt, das mit der erfindungsgemäßen Lager- und Mischvorrichtung durchgeführt wird. Die fertig aufgebaute Lager- und Mischvorrichtung wird so, wie sie in den Figuren 1 und 2 gezeigt ist, bereitgestellt. Die Glasampulle 8 wird geöffnet, indem der Ampullenkopf 24 abgebrochen wird, so dass die Monomerflüssigkeit 42 aus der Glasampulle 8 durch den Filter 28 und/oder das Sieb 28 und die Öffnung 30 in die Aufnahme 16 sowie in den unteren Bereich der Fluidleitung 6 hinein fließt. Dabei werden Bruchstücke des Glases und der abgebrochene Ampullenkopf 24 durch das Sieb 28 und/oder den Filter 28 zurückgehalten. Die Monomerflüssigkeit 42 aus der Glasampulle 8 ist dann in die Aufnahme 16 und die Fluidleitung 6 eingefüllt und steht zur Anwendung bereit. Diese Situation ist in Figur 3 gezeigt.

Um im Innenraum der Kartusche 1 einen Unterdruck zur Vermeidung von Gaseinschlüssen im Knochenzementteig 44 (siehe Figur 6) zu erzeugen, und auch um die Monomerflüssigkeit 42 in den Innenraum der Kartusche 1 zu transferieren, wird der Kolben 2 mit Hilfe des Mischstabs 9 in Richtung der Rückseite der Kartusche 1 gedrückt. Dabei vergrößert sich das Volumen zwischen dem Kolben 2 und dem Kartuschenkopf 3 im Innenraum der Kartusche 1, so dass ein Unterdruck entsteht. Die Luft zwischen dem Kolben 2 und dem Pumpkolben 15 kann nach außen austreten, so dass sich zwischen dem Kolben 2 und dem Pumpkolben 15 kein Überdruck aufbaut. Der Kolben 2 trifft, nachdem er die Distanz zwischen dem Kolben 2 und dem Pumpkolben 15 überwunden hat, auf dem Pumpkolben 15 und drückt diesen zur Einmündung der Fluidleitung 6 in die Aufnahme 16. Dabei schließt sich die Öffnung 30 und die Monomerflüssigkeit 42 wird aus der Aufnahme 16 in die Fluidleitung 6 gedrückt und später durch die Fluidleitung 6 und die Durchführung 5 in den Innenraum der Kartusche 1 zwischen dem Kolben 2 und dem Kartuschenkopf 3. Zudem saugt der Unterdruck die Monomerflüssigkeit 42 aus der Fluidleitung 6 in die Kartusche 1 und schließlich über die Durchführung 5 und den Porenfilter 30 in die Kartusche 1 hinein (siehe Figur 4). Der Kolben 2 ist zu Beginn (siehe Figur 1) derart weit von der Rückseite beziehungsweise dem Anschlag 18 beabstandet, dass der vollständige Hub des Kolbens 2 bis zum Anschlag 18 dazu ausreicht, dass sich der Druck im Innenraum der Kartusche 1 zwischen dem Kolben 2 und dem Kartuschenkopf 3 zumindest halbiert. Der Pumpkolben 15 wird bis zum Anschlag in die Aufnahme 16 eingedrückt, um die gesamte Monomerflüssigkeit 42 aus der Aufnahme 16 heraus zu drücken. Es verbleibt eine Restmenge der Monomerflüssigkeit 42 in der Fluidleitung 6.

Wenn die Monomerflüssigkeit 42 mit dem Pumpkolben 15 in den Innenraum der Kartusche 1 gedrückt ist, kann sie mit Hilfe der Mischeinrichtung 14 mit dem Knochenzementpulver 4 gemischt werden, indem die Mischeinrichtung 14 mit Hilfe des Mischtabs 9 manuell in Längsrichtung axial bewegt und im Innenraum der Kartusche 1 gedreht wird. Dabei entsteht der fertig gemischte Knochenzementteig 44 (siehe Figur 6). Anschließend wird die Mischeinrichtung 14 mit Hilfe des Mischstabs 9 bis zum Anschlag an den Kartuschenkopf 3 gezogen, gegebenenfalls eine Sicherung (nicht gezeigt) entfernt und der Mischstab 9 mit der Überwurfmutter 34 am Kartuschenkopf 3 fixiert. Der Kern 10 wird mit Hilfe des Griffs 12 aus dem Mischstab 9 herausgezogen. Der Mischstab 9 bildet nun ein in der Austragsöffnung angeordnetes beziehungsweise durch die Austragsöffnung geführtes Austragsrohr 9, durch das der fertig gemischte Knochenzementteig 44 appliziert werden kann.

Hierzu wird die Kartusche 1 vom Fußteil 20 getrennt, indem sie herausgezogen wird und die Fluidleitung 6 von der Durchführung 5 abgezogen wird. In der Fluidleitung 6 kann ein Rückschlagventil (nicht gezeigt) vorgesehen sein, um ein Austreten der restlichen Monomerflüssigkeit 42 aus der Fluidleitung 6 zu verhindern. Die Kartusche 1 wird dann in eine Auspressvorrichtung (nicht gezeigt) eingesetzt, mit der der Kolben 2 als Austragskolben 2 mit Hilfe einer vortreibbaren Stange in Richtung des Kartuschenkopfs 3 gepresst werden kann. Dabei wird der Knochenzementteig 44 durch das Austragsrohr 9 und durch die Austragsöffnung ausgepresst. Durch die Durchführung 5 kann kein Knochenzementteig 44 entweichen, da der Porenfilter 36 den Knochenzementteig 44 zurückhält.

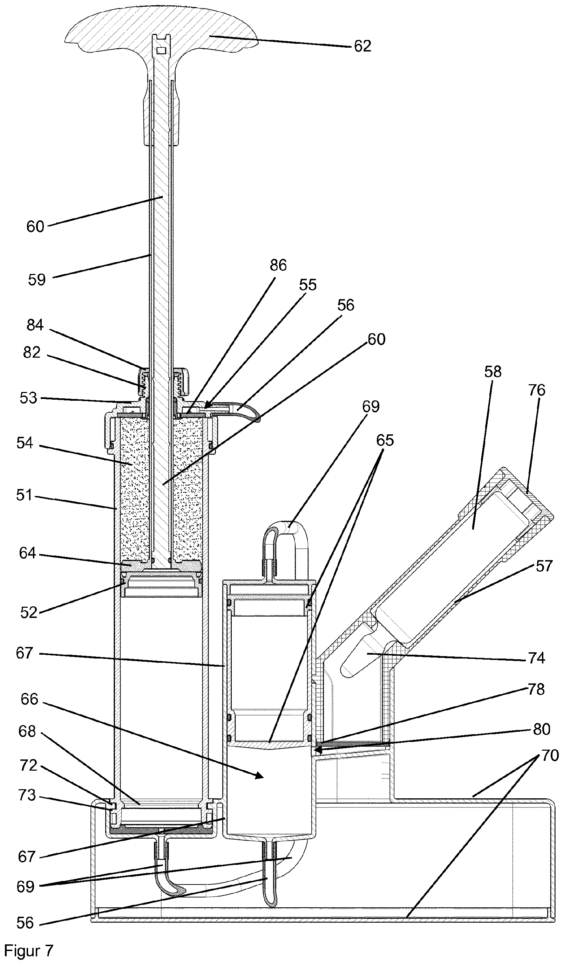

Die Figuren 7 bis 13 zeigen eine beispielhafte Ausführung einer alternativen zweiten erfindungsgemäßen Lager- und Mischvorrichtung. Figur 7 zeigt dabei eine schematische Querschnittansicht der ersten beispielhaften erfindungsgemäßen Lager- und Mischvorrichtung im Ausgangszustand. Die Lager- und Mischvorrichtung umfasst eine Kartusche 51 mit einem zylindrischen Innenraum, in dem ein Kolben 52 linear axial beweglich angeordnet ist. An der Vorderseite der Kartusche 51 (Figuren 7 und 9 bis 13 oben) ist der Innenraum beziehungsweise die Kartusche 51 durch einen Kartuschenkopf 53 nach Art eines Deckels oder einer Kappe verschlossen. Der Kartuschenkopf 53 ist gegen die Kartusche 51 mit einer umlaufenden Dichtung abgedichtet. In dem Innenraum der Kartusche 51 befindet sich zwischen dem Kolben 52 und dem Kartuschenkopf 53 ein Knochenzementpulver 54 als erste Ausgangskomponente 54 eines PMMA-Knochenzements.

Der Kolben 52 schließt mit einer umlaufenden Gummidichtung gasdicht mit der Innenwand des Innenraums der Kartusche 51 ab. Dadurch kann durch eine Bewegung des Kolbens 52 vom Kartuschenkopf 53 weg im Innenraum der Kartusche 51 zwischen dem Kolben 52 und dem Kartuschenkopf 53 ein Unterdruck entstehen, der durch eine Durchführung 55 im Kartuschenkopf 53 zu einer Fluidleitung 56 geleitet wird, beziehungsweise auf die Fluidleitung 56 einwirkt, und zwischen dem Kolben 52 und einem Verschluss an der Rückseite der Kartusche 51 ein Überdruck entstehen oder ein Druck auf eine darin enthaltene Hydraulikflüssigkeit (nicht gezeigt) ausgeübt werden.

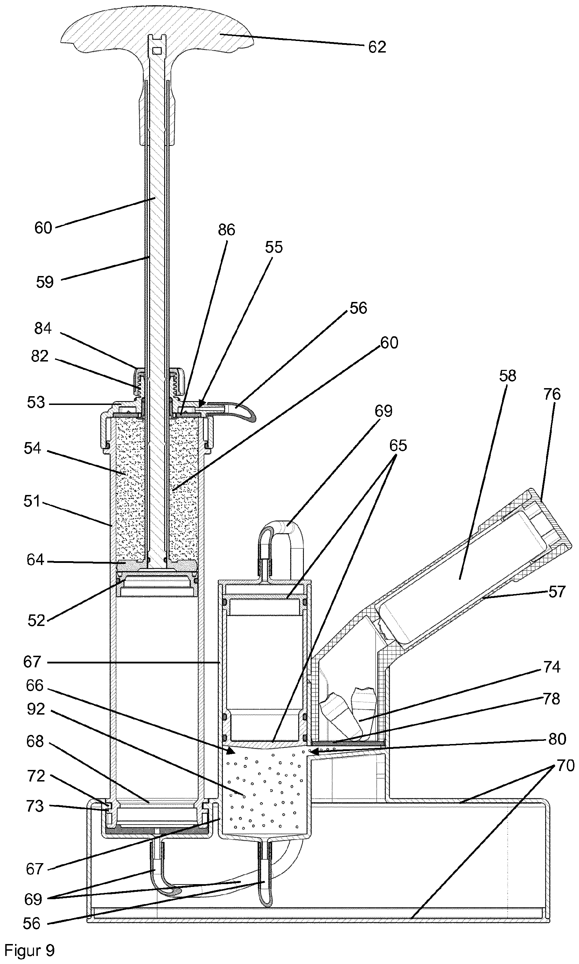

Ein zur Kartusche 51 separater Behälter 57 ist neben der Kartusche 51 angeordnet. In dem Behälter 57 ist eine Glasampulle 58 kopfüber eingesteckt. Die Glasampulle 58 ist in den geschnittenen Ansichten der Figuren 7 und 9 bis 12 nicht geschnitten dargestellt und ist in Figur 7 mit einer Monomerflüssigkeit 92 (siehe Figuren 9 bis 11) gefüllt.

In dem Kartuschenkopf 53 befindet sich eine zentrale Austragsöffnung, durch die ein hohler beziehungsweise rohrförmiger Mischstab 59 hindurchgeführt ist. Der hohle Mischstab 59 ist in seinem Inneren mit einem Kern 60 verschlossen, der sich aus dem Mischstab 59 herausziehen lässt. Der Mischstab 59 ist mit einer umlaufenden Dichtung im Bereich der Austragsöffnung gegen den Kartuschenkopf 53 abgedichtet, so dass der Unterdruck im Innenraum der Kartusche 51 nicht (oder zumindest nicht schnell) Luft zwischen dem Kartuschenkopf 53 und dem Mischstab 59 in den Innenraum der Kartusche 51 nachziehen kann, auch wenn der Mischstab 59 in der Austragsöffnung gedreht und in Längsrichtung bewegt wird.

An dem vorderen Ende des Mischstabs 59 ist ein Griff 62 angeordnet, mit dem der Mischstab 59 manuell gegen die Kartusche 51 in Längsrichtung bewegt und axial gedreht werden kann. Zudem kann mit dem Griff 62 nach Lösen einer Rastung (nicht gezeigt) oder nach Überwinden eines Widerstands der Kern 60 aus dem Mischstab 59 herausgezogen werden.

Am Mischstab 59 ist im Innenraum der Kartusche 51 zwischen dem Kolben 52 und dem Kartuschenkopf 53 eine Mischeinrichtung 64 mit mehreren Mischflügeln 64 befestigt, so dass sich beim Drehen und Bewegen des Mischstabs 59 gegen die Kartusche 51 auch die Mischeinrichtung 64 im Innenraum der Kartusche 51 dreht und axial bewegt. Damit kann über den Mischstab 59 der Inhalt des Innenraums der Kartusche 51 mit Hilfe der Mischeinrichtung 64 mechanisch durchmischt werden.

Ein Pumpkolben 65 ist oberhalb einer Aufnahme 66 in einem Hohlzylinder 67 angeordnet. Der Hohlzylinder 67 ist neben der Kartusche 51 angeordnet und begrenzt die Aufnahme 66 seitlich und nach unten, wobei der Pumpkolben 65 die Aufnahme 66 nach oben begrenzt. Damit der Kolben 52 nicht bodenseitig aus dem Innenraum der Kartusche 51 herausgedrückt werden kann, ist an der Innenseite der Kartusche 51 im Bereich der Rückseite des Innenraums ein Anschlag 68 in Form einer umlaufenden Erhebung vorgesehen. Der Kolben 52 kann also nur bis zum Anschlag 68 in Richtung der Rückseite der Kartusche 51 gedrückt werden. Nach dem Entfernen der Kartusche 51 aus der Lager- und Mischvorrichtung wird mit dem Anschlag 68 sichergestellt, dass der Kolben 52 nicht aus der Rückseite der Kartusche 51 austreten kann.

Die Rückseite der Kartusche 51 ist druckdicht verschlossen und eine Gasleitung 69 oder Hydraulikleitung 69 ist am Boden des Verschlusses angeschlossen, die die Rückseite der Kartusche 51 mit der Oberseite des Hohlzylinders 67 verbindet. Der Pumpkolben 65 schließt mit Hilfe dreier umlaufender Dichtungen druckdicht mit den Innenwänden des Hohlzylinders 67 ab. Der Pumpkolben 65 ist in dem durch den Hohlzylinder 67 gebildeten zylindrischen Hohlraum in Längsrichtung beweglich gelagert. An der Unterseite der Aufnahme 66 mündet die Fluidleitung 56 in die Aufnahme 66.

Die Fluidleitung 56 erstreckt sich bereichsweise parallel zur Achse der zylindrischen Kartusche 51 nach unten vom Kartuschenkopf 53 weg, vollführt dann eine Kurve und erstreckt sich dann bis zur Einmündung in die Aufnahme 66 nach oben. Die Aufnahme 66 könnte auch als Verbreiterung der Fluidleitung 56 aufgefasst werden. Die Aufnahme 66 bildet ein Reservoir für die Monomerflüssigkeit 92 aus der Glasampulle 58.

Um im Innenraum der Kartusche 51 einen Unterdruck zu erzeugen, kann der Kolben 52 in Richtung einer Rückseite der Kartusche 51 (bodenseitig, das heißt in den Figuren 7 bis 12 nach unten) beziehungsweise in Richtung der Rückseite des Innenraums der Kartusche 51 bewegt werden, indem der Kolben 52 mit Hilfe des Mischstabs 59 manuell in Richtung der Rückseite gedrückt wird.

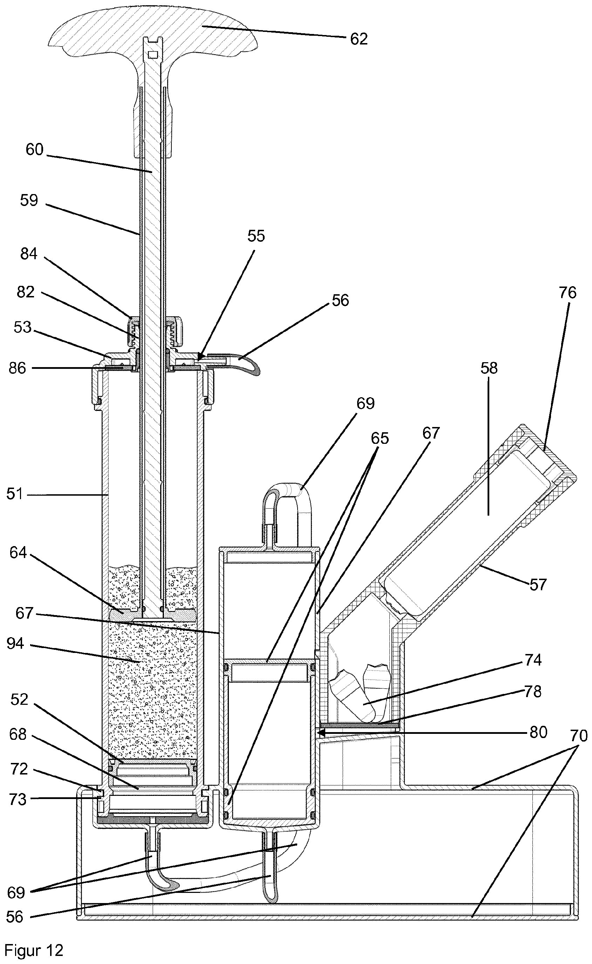

Die Kartusche 51 ist lösbar mit einem Fußteil 70 verbunden. An der Rückseite der Kartusche 51 sind zur Befestigung mit dem Fußteil 70 außen zwei umlaufende Vorsprünge 72 angeordnet. Hierzu wird die Kartusche 51 bodenseitig, also im Bereich der Rückseite mit einer Halterung 73 mit dem Fußteil 70 gerastet. Diese Vorsprünge 72 können zudem zur Befestigung eine Auspressvorrichtung (nicht gezeigt) verwendet werden, mit der der fertig gemischte Knochenzementteig 94 (siehe Figur 12) appliziert werden kann. Dazu wird der Kolben 52 als Austragskolben 52 mit der Auspressvorrichtung durch eine vortreibbare Stange in die Kartusche 51 in Richtung des Kartuschenkopfs 53 eingepresst und dabei der Knochenzementteig 94 durch das Austragsrohr 59 beziehungsweise den hohlen Mischstab 59 ausgepresst.

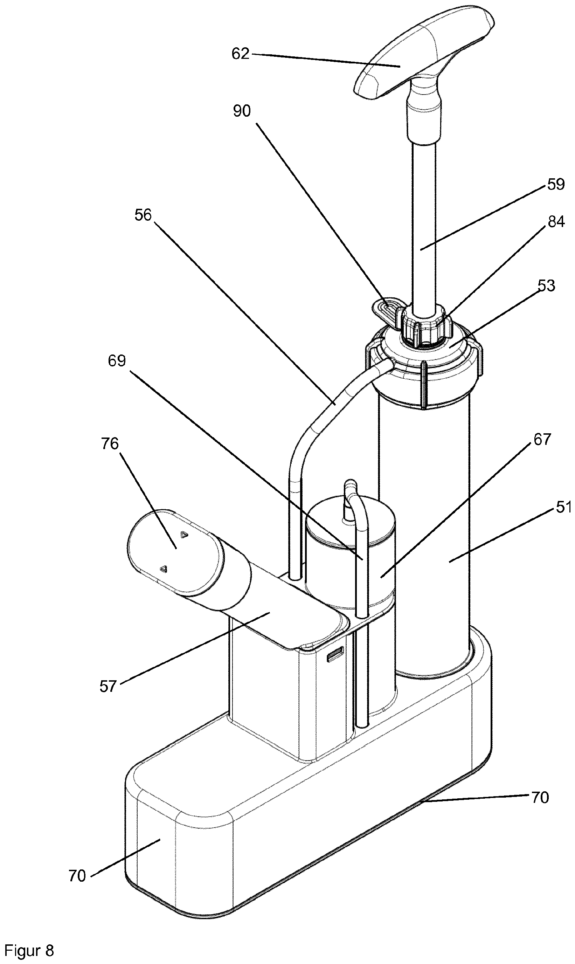

Die Glasampulle 58 weist einen abbrechbaren Ampullenkopf 74 auf. Wenn der Ampullenkopf 74 abgebrochen oder aufgebrochen wird, wird damit die Glasampulle 58 geöffnet. Der Behälter 57 ist aus einem elastisch verformbaren Material gefertigt, das eine Verdickung am Hals zwischen dem Ampullenkopf 74 und dem Körper der Ampulle 58 aufweist. Dadurch ist der Ampullenkopf 74 fest gelagert, während der Körper der Ampulle 58 aufgrund der Elastizität des den Behälter 57 bildenden Einsatzes bewegt werden kann. So kann die Glasampulle 58 innerhalb der Lager- und Mischvorrichtung aufgebrochen werden. An der Rückseite des Behälters 57 ist ein Stopfen 76 als Deckel 76 vorgesehen, mit dem die Glasampulle 58 in Position gehalten wird. In dem Deckel 76 sind zwei Öffnungen (siehe Figur 8) vorgesehen, durch die Luft ins Innere des Behälters 57 nachströmen kann.

In der Verbindung zwischen dem Behälter 57 und der Aufnahme 66 beziehungsweise unterhalb des Behälters 57 ist ein Filter 78 und/oder Sieb 78 angeordnet, mit dem Bruchstücke beziehungsweise Glassplitter der Glasampulle 58, die beim Aufbrechen der Glasampulle 58 entstehen können, zurückgehalten werden. Unterhalb der Filters 78 und/oder Siebs 78 neigt sich der Boden und der Behälter 57 und die Aufnahme 66 sind über eine Öffnung 80 flüssigkeitsdurchlässig miteinander verbunden. Nach dem Öffnen der Glasampulle 58 fließt die Monomerflüssigkeit 92 nach unten ab über eine geneigte Fläche und durch die Öffnung 80 in die Aufnahme 66 hinein (siehe Figur 9). Der abgebrochene Ampullenkopf 74 und eventuell entstehende Glassplitter werden dabei von dem Sieb 78 und/oder dem Filter 78 zurückgehalten.

Im Bereich der Austragsöffnung ist am Kartuschenkopf 53 ein Stutzen 82 mit einem Außengewinde vorgesehen. Auf dieses Außengewinde kann eine konische Überwurfmutter 84 als Befestigungseinrichtung 84 aufgeschraubt werden, um den Mischstab 59 gegen den Kartuschenkopf 53 zu fixieren. Um den Mischstab 59 gegen den Kartuschenkopf 53 fixieren zu können, wird die Überwurfmutter 84 weiter auf das Außengewinde des Stutzens 82 geschraubt. Um dies zunächst zu verhindern, beziehungsweise um eine ungewollte Fixierung zu vermeiden, kann eine herausziehbare Sicherung (nicht gezeigt) in Form eine Spange mit einer Greiflasche vorgesehen, die zwischen der Überwurfmutter 84 und dem Kartuschenkopf 53 angeordnet ist. Wenn die Sicherung abgezogen wird, kann die Überwurfmutter 84 weiter auf das Außengewinde des Stutzens 82 geschraubt werden, so dass der Stutzen 82 zusammengedrückt wird und dadurch den Mischstab 59 gegen den Kartuschenkopf 53 fixiert.

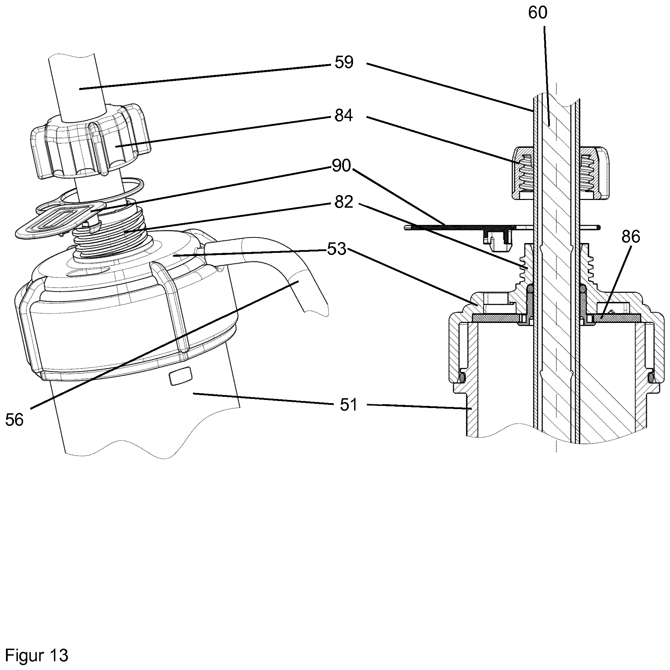

Eine zusätzliche seitliche Öffnung ist im Kartuschenkopf 53 vorgesehen, durch die der Innenraum der Kartusche 51 für sterilisierende Gase, wie Ethylenoxid, zugänglich ist. Vorliegend ist die seitliche Öffnung mit einem Verschluss 90 verschlossen. Der Verschluss 90 ist in geöffneter Stellung in Figur 13 gezeigt. Im geöffneten Zustand nach Figur 13 kann der Innenraum der Kartusche 51 durch die seitlich neben der Austragsöffnung angeordnete Öffnung im Kartuschenkopf 53 sterilisiert werden, indem ein sterilisierendes Gas, wie beispielsweise Ethylenoxid, eingespeist wird. Anschließend kann der Verschluss 40 in die Öffnung hinein gedrückt werden, um diese zu verschließen. Danach wird die Überwurfmutter 84 so weit auf den Stutzen 82 geschraubt, dass der Mischstab 59 gegen die Kartuschenkopf 53 abgedichtet ist, aber noch frei in der Austragsöffnung beweglich ist.

Zwischen dem Kartuschenkopf 53 und dem Innenraum der Kartusche 51 ist eine Porenscheibe 86 beziehungsweise ein Porenfilter 86 angeordnet, der für Gase und Flüssigkeiten durchlässig ist aber für Pulver und Feststoffe wie das Knochenzementpulver 54 undurchlässig ist, so dass die Durchführung 55 nur über den Porenfilter 86 mit dem restlichen Innenraum der Kartusche 51 verbunden ist. Der Porenfilter 86 verhindert ein Vordringen des Knochenzementpulvers 54 in die Fluidleitung 56 und damit eine ungewollte vorzeitige Reaktion des Knochenzementpulvers 54 mit der Monomerflüssigkeit 92 und damit einen ungewollten Verschluss des dünnen Teils der Fluidleitung 56.

Anhand der Figuren 7 bis 12 wird im Folgenden ein exemplarisches erfindungsgemäßes Verfahren vorgestellt, das mit der erfindungsgemäßen Lager- und Mischvorrichtung durchgeführt wird. Die fertig aufgebaute Lager- und Mischvorrichtung wird so, wie sie in den Figuren 7 und 8 gezeigt ist, bereitgestellt. Die Glasampulle 58 wird geöffnet, indem der Ampullenkopf 74 abgebrochen wird, so dass die Monomerflüssigkeit 92 aus der Glasampulle 58 durch den Filter 78 und/oder das Sieb 78 und die Öffnung 80 in die Aufnahme 66 sowie in den unteren Bereich der Fluidleitung 56 hinein fließt. Dabei werden Bruchstücke des Glases und der abgebrochene Ampullenkopf 74 durch das Sieb 78 und/oder den Filter 78 zurückgehalten. Die Monomerflüssigkeit 92 aus der Glasampulle 58 ist dann in die Aufnahme 66 und die Fluidleitung 56 eingefüllt und steht zur Anwendung bereit. Diese Situation ist in Figur 9 gezeigt.

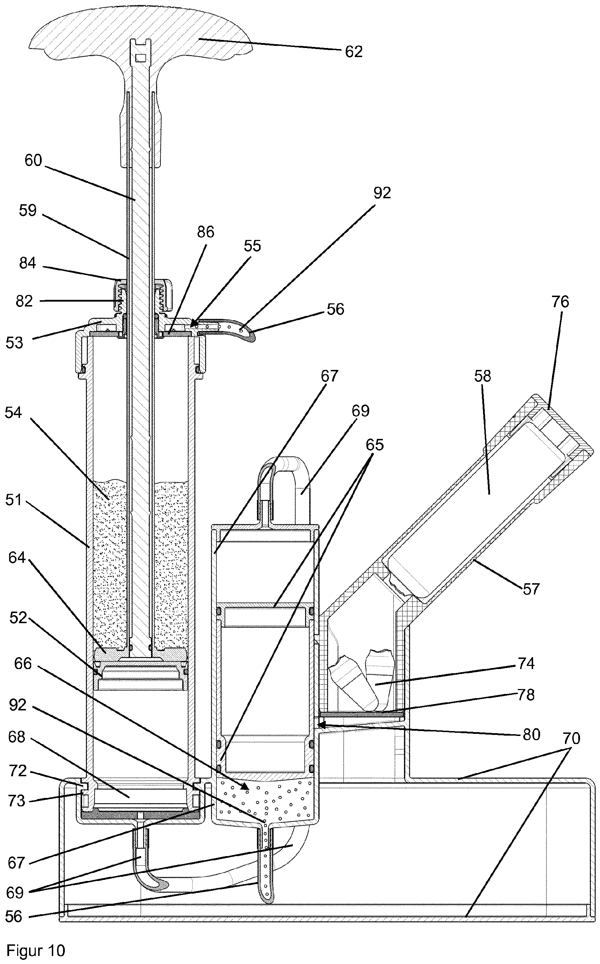

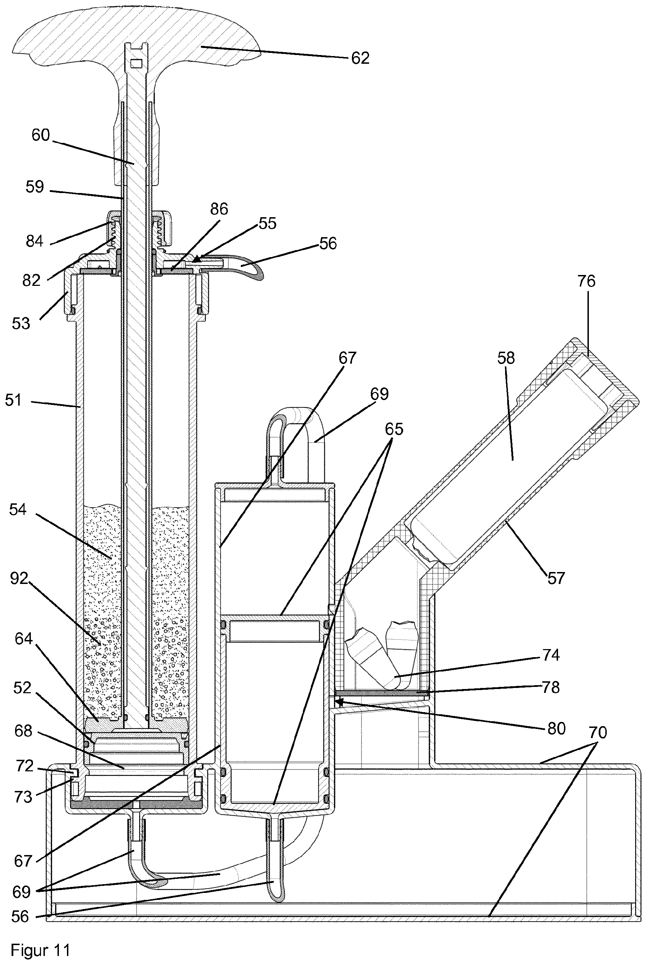

Um im Innenraum der Kartusche 51 einen Unterdruck zur Vermeidung von Gaseinschlüssen im Knochenzementteig 94 (siehe Figur 12) zu erzeugen, und auch um die Monomerflüssigkeit 92 in den Innenraum der Kartusche 51 zu transferieren, wird der Kolben 52 mit Hilfe des Mischstabs 59 in Richtung der Rückseite der Kartusche 51 gedrückt. Dabei vergrößert sich das Volumen zwischen dem Kolben 52 und dem Kartuschenkopf 53 im Innenraum der Kartusche 51, so dass ein Unterdruck entsteht. Gleichzeitig entsteht zwischen dem Kolben 52 und der verschlossenen Rückseite der Kartusche 51 ein Überdruck, der durch die Gasleitung 69 oben in den Hohlzylinder 67 zwischen die Oberseite des Hohlzylinders 67 und den Pumpkolben 65 eingeleitet wird, oder ein hydrostatischer Druck, der durch die Hydraulikleitung 69 oben in den Hohlzylinder 67 zwischen die Oberseite des Hohlzylinders 67 und den Pumpkolben 65 geleitet wird. Durch den Überdruck oder den hydrostatischen Druck wird der Pumpkolben 65 im Hohlzylinder 67 nach unten zur Einmündung der Fluidleitung 56 in die Aufnahme 66 gedrückt. Dabei schließt sich die Öffnung 80 und die Monomerflüssigkeit 92 wird aus der Aufnahme 66 in die Fluidleitung 56 gedrückt und später durch die Fluidleitung 56 und die Durchführung 55 in den Innenraum der Kartusche 51 zwischen dem Kolben 52 und dem Kartuschenkopf 53. Zudem saugt der Unterdruck die Monomerflüssigkeit 92 aus der Fluidleitung 56 in die Kartusche 51 und schließlich über die Durchführung 55 und den Porenfilter 80 in die Kartusche 51 hinein (siehe Figuren 10 und 11). Der Kolben 52 ist zu Beginn der Bewegung (siehe Figur 7) derart weit von der Rückseite beziehungsweise dem Anschlag 68 beabstandet, dass der vollständige Hub des Kolbens 52 bis zum Anschlag 68 dazu ausreicht, dass sich der Druck (Gasdruck) im Innenraum der Kartusche 51 zwischen dem Kolben 52 und dem Kartuschenkopf 53 zumindest halbiert und dass der Überdruck, der sich zwischen dem Kolben 52 und der verschlossenen Rückseite der Kartusche 51 aufbaut dazu ausreicht, den Pumpkolben 65 im Hohlzylinder 67 zu drücken, oder dass das Volumen des Hubs dem Volumen des Hubs des Pumpkolbens 65 entspricht.

Da der Raum zwischen dem Kolben 52 und der verschlossenen Rückseite der Kartusche 51, in der Gasleitung 69 oder der Hydraulikleitung 69 und zwischen dem Pumpkolben 65 und der Oberseite des Hohlzylinders 67 abgeschlossen ist, kann vorgesehen sein, dass in diesem abgeschlossenen Raum eine Hydraulikflüssigkeit (nicht gezeigt) vorgesehen ist, mit der der Druck hydraulisch übertragen durch die Hydraulikleitung 69 werden kann.

Der Pumpkolben 65 wird bis zum Anschlag in die Aufnahme 66 eingedrückt, um die gesamte Monomerflüssigkeit 92 aus der Aufnahme 66 heraus zu drücken. Es verbleibt eine Restmenge der Monomerflüssigkeit 92 in der Fluidleitung 56.

Wenn die Monomerflüssigkeit 92 mit dem Pumpkolben 65 in den Innenraum der Kartusche 51 gedrückt ist, kann sie mit Hilfe der Mischeinrichtung 64 mit dem Knochenzementpulver 54 gemischt werden, indem die Mischeinrichtung 54 mit Hilfe des Mischtabs 59 manuell in Längsrichtung axial bewegt und im Innenraum der Kartusche 51 gedreht wird. Dabei entsteht der fertig gemischte Knochenzementteig 94 (siehe Figur 12). Anschließend wird die Mischeinrichtung 64 mit Hilfe des Mischstabs 59 bis zum Anschlag an den Kartuschenkopf 53 gezogen, gegebenenfalls eine Sicherung (nicht gezeigt) entfernt und der Mischstab 59 mit der Überwurfmutter 84 am Kartuschenkopf 53 fixiert. Der Kern 60 wird mit Hilfe des Griffs 62 aus dem Mischstab 59 herausgezogen. Der Mischstab 59 bildet nun ein in der Austragsöffnung angeordnetes beziehungsweise durch die Austragsöffnung geführtes Austragsrohr 59, durch das der fertig gemischte Knochenzementteig 94 appliziert werden kann.

Hierzu wird die Kartusche 51 vom Fußteil 70 getrennt, indem sie herausgezogen wird und die Fluidleitung 56 von der Durchführung 55 abgezogen wird. In der Fluidleitung 56 kann ein Rückschlagventil (nicht gezeigt) vorgesehen sein, um ein Austreten der restlichen Monomerflüssigkeit 92 aus der Fluidleitung 56 zu verhindern. Die Kartusche 51 wird dann in eine Auspressvorrichtung (nicht gezeigt) eingesetzt, mit der der Kolben 52 als Austragskolben 52 mit Hilfe einer vortreibbaren Stange in Richtung des Kartuschenkopfs 53 gepresst werden kann. Dabei wird der Knochenzementteig 94 durch das Austragsrohr 59 und durch die Austragsöffnung ausgepresst. Durch die Durchführung 55 kann kein Knochenzementteig 94 entweichen, da der Porenfilter 86 den Knochenzementteig 94 zurückhält.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 51: Kartusche
- 2, 52: Kolben / Austragskolben
- 3, 53: Kartuschenkopf
- 4, 54: Knochenzementpulver / erste Ausgangskomponente
- 5, 55: Durchführung
- 6, 56: Fluidleitung
- 7, 57: Behälter
- 8, 68: Monomerflüssigkeitsbehälter / Glasampulle
- 9, 59: Mischstab / Austragsrohr
- 10, 60: Kern
- 12, 62: Griff
- 14, 64: Mischflügel / Mischeinrichtung
- 15, 65: Pumpkolben
- 16, 66: Aufnahme
- 18, 68: Anschlag
- 20, 70: Fußteil
- 22, 72: Vorsprung
- 23, 73: Halterung
- 24, 74: Ampullenkopf
- 26, 76: Deckel / Stopfen
- 28, 78: Sieb / Filter
- 30, 80: Öffnung
- 32, 82: Stutzen mit Außengewinde
- 34, 84: konische Überwurfmutter / Befestigungseinrichtung
- 36, 86: Porenfilter / Porenscheibe
- 40, 90: Verschluss
- 42, 92: Monomerflüssigkeit
- 44, 94: gemischter Knochenzementteig
- 46: Dichtung
- 67: Hohlzylinder
- 69: Gasleitung / Hydraulikleitung

## Patentansprüche

1. Lager- und Mischvorrichtung für Zweikomponenten-Polymethylmethacrylat-Knochenzemente, die Lager- und Mischvorrichtung aufweisend eine Kartusche (1, 51) mit einem zylindrischen Innenraum, wobei der zylindrische Innenraum an einer Vorderseite durch einen Kartuschenkopf (3, 53) mit einer geschlossenen Austragsöffnung begrenzt ist,
einen Kolben (2, 52), der axial beweglich im zylindrischen Innenraum der Kartusche (1, 51) angeordnet ist und der von dem Kartuschenkopf (3, 53) beabstandet ist, wobei der Kolben (2, 52) umlaufend an der Innenwand des Innenraums anliegt, so dass der Kolben (2, 52) den Innenraum der Kartusche (1, 51) in zwei Bereiche trennt,
eine pulverförmige erste Ausgangskomponente (4, 54) des Knochenzements, die in dem Innenraum zwischen dem Kolben (2, 52) und dem Kartuschenkopf (3, 53) enthalten ist,
eine Durchführung (5, 55), die im Kartuschenkopf (3, 53) oder in dem Zylindermantel der Kartusche (1, 51) zwischen dem Kolben (2, 52) und dem Kartuschenkopf (3, 53) angeordnet ist, wobei die Durchführung (5, 55) mit einer Fluidleitung (6, 56) verbunden ist,
eine Aufnahme (16, 66) für eine Monomerflüssigkeit (42, 92), wobei die Fluidleitung (6, 56) die Durchführung (5, 55) mit der Aufnahme (16, 66) flüssigkeitsdurchlässig verbindet, wobei die Monomerflüssigkeit (42, 92) als zweite Ausgangskomponente des Knochenzements in der Aufnahme (16, 66) enthalten ist oder in die Aufnahme (16, 66) einfüllbar oder einleitbar ist,
eine Ausdrückvorrichtung (15, 65), die in die Aufnahme (16, 66) hinein zu drücken ist, so dass Monomerflüssigkeit (42, 92) aus der Aufnahme (16, 66) in die Fluidleitung (6, 56) hinein drückbar ist, **dadurch gekennzeichnet, dass** der Kolben (2, 52) von einer der Vorderseite gegenüberliegenden Rückseite des zylindrischen Innenraums beabstandet ist, so dass durch eine Bewegung des Kolbens (2, 52) in Richtung der Rückseite die Ausdrückvorrichtung (15, 65) in die Aufnahme (16, 66) hinein zu drücken ist und ein Unterdruck in dem Innenraum der Kartusche (1, 51) zwischen dem Kolben (2, 52) und dem Kartuschenkopf (3, 53) erzeugbar ist.

2. Lager- und Mischvorrichtung nach Anspruch 1, **dadurch gekennzeichnet, dass** der Kolben (2, 52) mit einem Stab (9, 59) oder mit einem anderen Kraftübertragungsmittel in dem Innenraum der Kartusche (1, 51) manuell in Richtung der Rückseite des Innenraums zu drücken oder zu ziehen ist, wobei vorzugsweise an dem Stab (9, 59) oder an dem Kraftübertragungsmittel ein Betätigungsmittel oder ein Griff (12, 62) zum Bedienen des Stabs (9, 59) oder des Kraftübertragungsmittels befestigt ist.

3. Lager- und Mischvorrichtung nach Anspruch 1 oder 2, **dadurch gekennzeichnet, dass**
im Kartuschenkopf (3, 53), in der Durchführung (5, 55) und/oder in der Fluidleitung (6, 56) ein Filter (36, 86), insbesondere ein Porenfilter (36, 86), angeordnet ist, wobei der Filter (36, 86) für die Monomerflüssigkeit (42, 92) durchlässig und für die pulverförmige erste Ausgangskomponente (4, 54) undurchlässig ist und/oder
der Kolben (2, 52) von einer der Vorderseite gegenüberliegenden Rückseite des zylindrischen Innenraums derart weit beabstandet ist, dass mit dem Unterdruck die Monomerflüssigkeit (42, 92) aus der Fluidleitung (6, 56) in den Innenraum der Kartusche (1, 51) zu saugen ist.

4. Lager- und Mischvorrichtung nach einem der vorangehenden Ansprüche, **dadurch gekennzeichnet, dass**
die Lager- und Mischvorrichtung einen von der Kartusche (1, 51) und von der Aufnahme (16, 66) separaten Behälter (7, 57) aufweist, in dem die Monomerflüssigkeit (42, 92) enthalten ist, wobei der Behälter (7, 57) mit der Aufnahme (16, 66) verbunden ist, insbesondere über eine Öffnung (30, 80) verbunden ist, wobei vorzugsweise in dem Behälter (7, 57) eine geschlossene Glasampulle (8, 58) oder ein Folienbeutel enthaltend die Monomerflüssigkeit (42, 92) angeordnet oder anzuordnen ist, wobei die Glasampulle (8, 58) innerhalb des Behälters (7, 57) aufbrechbar ist oder der Folienbeutel innerhalb des Behälters (7, 57) aufzustechen oder aufzureißen ist.

5. Lager- und Mischvorrichtung nach Anspruch 4, **dadurch gekennzeichnet, dass** die Kartusche (1, 51), die Aufnahme (16, 66), der separate Behälter (7, 57) und die Fluidleitung (6, 56) mit einem gemeinsamen Standfuß (20, 70) verbunden sind, wobei die Aufnahme (16, 66), der Behälter (7, 57) und die Fluidleitung (6, 56) fest mit dem Standfuß (20, 70) verbunden sind und die Kartusche (1, 51) lösbar mit dem Standfuß (20, 70) verbunden ist, bevorzugt die Kartusche (1, 51) über ein Gewinde an den Standfuß (20, 70) geschraubt ist oder über eine Rastung (22, 23, 72, 73) mit dem Standfuß (20, 70) verbunden ist und/oder in dem separaten Behälter (7, 57) ein Monomerflüssigkeitsbehälter (8, 58) enthalten ist, insbesondere ein Folienbeutel oder eine Glasampulle (8, 58) enthalten ist, der oder die innerhalb des separaten Behälters (7, 57) zu öffnen ist, so dass die Monomerflüssigkeit (42, 92) aus dem geöffneten Monomerflüssigkeitsbehälter (8, 58) in die Aufnahme (16, 66) fließt, wobei vorzugsweise am Behälter (7, 57) eine von außen bedienbare Öffnungsvorrichtung zum Öffnen des Monomerflüssigkeitsbehälters (8, 58) angeordnet ist.

6. Lager- und Mischvorrichtung nach einem der vorangehenden Ansprüche, **dadurch gekennzeichnet, dass**
in dem Innenraum der Kartusche (1, 51) zwischen dem Kolben (2, 52) und dem Kartuschenkopf (3, 53) eine Mischeinrichtung (14, 64) angeordnet ist, mit der die erste Ausgangskomponente (4, 54) mit der Monomerflüssigkeit (42, 92) im Innenraum der Kartusche (1, 51) zu mischen ist.

7. Lager- und Mischvorrichtung nach Anspruch 6, **dadurch gekennzeichnet, dass** die Mischeinrichtung (14, 64) über einen Mischstab (9, 59) von außerhalb der Kartusche (1, 51) bedienbar ist, wobei der Mischstab (9, 59) gasdicht durch den Kartuschenkopf (3, 53) geführt ist sowie drehbar und in axialer Richtung beweglich ist, vorzugsweise der Mischstab (9, 59) durch die Austragsöffnung geführt ist , wobei bevorzugt der Kolben (2, 52) mit dem Mischstab (9, 59) in Richtung der Rückseite des Innenraums der Kartusche (1, 51) zu drücken ist und/oder der Mischstab (9, 59) ein Austragsrohr (9, 59) ist, in dem ein manuell entfernbarer Kern (10, 60) angeordnet ist, so dass der gemischte Knochenzementteig (44, 94) aus dem Innenraum der Kartusche (1, 51) durch das Austragsrohr (9, 59) ohne den Kern (10, 60) auszutreiben ist.

8. Lager- und Mischvorrichtung nach einem der vorangehenden Ansprüche, **dadurch gekennzeichnet, dass**
im Innenraum der Kartusche (1, 51) an der Rückseite ein Anschlag (18, 68) vorgesehen ist, der die Bewegung des Kolbens (2, 52) in Richtung Rückseite begrenzt, und/oder
der Kolben (2, 52) mindestens ein Rastelement besitzt, das mit einer Gegenrastung an der Innenseite der Kartusche (1, 51) im Bereich der Rückseite des Innenraums lösbar zu rasten ist.

9. Lager- und Mischvorrichtung nach einem der vorangehenden Ansprüche, **dadurch gekennzeichnet, dass**
im Kartuschenkopf (3, 53) eine gasdurchlässige Öffnung, insbesondere eine verschließbare gasdurchlässige Öffnung, angeordnet ist, wobei eine für Gase durchlässige und für Feststoffpartikel undurchlässige poröse Scheibe (36, 86) zwischen der ersten Ausgangskomponente (4, 54) und der Öffnung angeordnet ist, wobei bevorzugt die poröse Scheibe (36, 86) im Kartuschenkopf (3, 53) angeordnet ist.

10. Lager- und Mischvorrichtung nach einem der vorangehenden Ansprüche, **dadurch gekennzeichnet, dass**
eine Einmündung (30, 80) zum Eintragen der Monomerflüssigkeit (42, 92) in die Aufnahme (16, 66) im Bereich der Ausdrückvorrichtung (15, 65) angeordnet ist, wobei die Einmündung (30, 80) durch das Eindrücken der Ausdrückvorrichtung (15, 65) zu Beginn der Bewegung der Ausdrückvorrichtung (15, 65) in die Aufnahme (16, 66) hinein verschlossen wird.

11. Lager- und Mischvorrichtung nach einem der vorangehenden Ansprüche, **dadurch gekennzeichnet, dass**
die Aufnahme (16) an der Rückseite der Kartusche (1) angeordnet ist und der Kolben (2) während der Bewegung in Richtung der Rückseite der Kartusche (1) die Ausdrückvorrichtung (15) in die Aufnahme (16) drückt, wobei vorzugsweise der Bereich zwischen der Ausdrückvorrichtung (15) und dem Kolben (2) nach außen geöffnet ist, wobei bevorzugt der Kolben (2) von der Ausdrückvorrichtung (15) beabstandet ist, so dass bei einer Bewegung des Kolbens (2) in Richtung der Rückseite der Kartusche (1) in einem ersten Abschnitt zunächst ein Unterdruck im Innenraum der Kartusche (1) aufgebaut wird, ohne dass die Ausdrückvorrichtung (15) bewegt wird, und in einem zweiten Abschnitt zusätzlich die Ausdrückvorrichtung (15) in die Aufnahme (16) gedrückt wird, und/oder
die Ausdrückvorrichtung (15) ein Pumpkolben (15) ist und die Aufnahme (16) ein Hohlzylinder ist, in dem der Pumpkolben (15) axial beweglich ist, wobei auf der Oberseite des Pumpkolbens (15) eine Verlängerung, insbesondere in Form eines zylinderförmigen Körpers, angeordnet ist, die einen Außendurchmesser kleiner oder gleich dem Innendurchmesser des Innenraums der Kartusche (1) hat, wobei der Pumpkolben (15) mit der Verlängerung derart in dem Hohlzylinder angeordnet ist, dass die Verlängerung in den Innenraum der Kartusche (1) ragt.

12. Lager- und Mischvorrichtung nach einem der Ansprüche 1 bis 10, **dadurch gekennzeichnet, dass**
die Aufnahme (66) durch einen zur Kartusche (51) separaten geschlossenen Hohlzylinder (67) begrenzt ist, in dem ein Pumpkolben (65) als Ausdrückvorrichtung (65) axial beweglich gelagert ist, wobei der Pumpkolben (65) umlaufend und dicht mit der Innenwand des Hohlzylinders (67) schließt, wobei der Hohlzylinder (67) an einer ersten, vorzugsweise unteren, Grundfläche mit der Fluidleitung (56) verbunden ist und an einer zweiten, vorzugsweise oberen, Grundfläche über eine Druckgasleitung (69) oder eine Hydraulikleitung (69) an der Rückseite der Kartusche (51) mit dem Innenraum der Kartusche (51) verbunden ist, wobei die Rückseite der Kartusche (51) bis auf die Einmündung in die Druckgasleitung (69) oder die Hydraulikleitung (69) verschlossen ist, so dass bei einer Bewegung des Kolbens (52) in Richtung der Rückseite des Innenraums in dem Raum zwischen dem Kolben (52) und der Rückseite ein Überdruck oder ein Druck entsteht, der durch die Druckgasleitung (69) oder die Hydraulikleitung (69) in den Hohlzylinder (67) geleitet wird.

13. Verfahren zur Vermischung der Ausgangskomponenten (4, 42, 54, 92) eines Knochenzements, insbesondere eines Zweikomponenten-Polymethylmethacrylat-Knochenzements, mit einer Lager- und Mischvorrichtung nach mindestens einem der vorangehenden Ansprüche, umfassend die folgenden Schritte,
a) der Kolben (2, 52) wird in Richtung der Rückseite des zylindrischen Innenraums der Kartusche (1, 51) bewegt, wobei durch die Bewegung des Kolbens (2, 52) im Innenraum der Kartusche (1, 51) zwischen dem Kolben (2, 52) und dem Kartuschenkopf (3, 53) ein Unterdruck entsteht,
b) durch die Bewegung des Kolbens (2, 52) wird die Ausdrückvorrichtung (15, 65) in die Aufnahme (16, 66) gedrückt, wodurch eine in der Aufnahme (16, 66) enthaltene Monomerflüssigkeit (42, 92) durch die Bewegung der Ausdrückvorrichtung (15, 65) durch die Fluidleitung (6, 56) in den Innenraum der Kartusche (1, 51) gedrückt wird,
c) die Monomerflüssigkeit (42, 92) und die erste Ausgangskomponente (4, 54) werden im Innenraum der Kartusche (1, 51) zu dem Knochenzementteig (44, 94) gemischt, und
d) der Knochenzementteig (44, 94) wird durch Vortreiben des Kolbens (2, 52) in Richtung des Kartuschenkopfs (3, 53) aus dem Innenraum der Kartusche (1, 51) ausgetrieben.

14. Verfahren nach Anspruch 13, **dadurch gekennzeichnet, dass**
die Monomerflüssigkeit (42, 92) mit dem Unterdruck im Innenraum der Kartusche (1, 51) aus der Fluidleitung (6, 56) in den Innenraum der Kartusche (1, 51) gesaugt wird und/oder
vor Schritt a) die Monomerflüssigkeit (42, 92) in die Aufnahme (16, 66) eingeleitet wird, insbesondere nach Öffnen eines Monomerflüssigkeitsbehälters (8, 58) in einem Behälter (7, 57) der Lager- und Mischvorrichtung.

15. Verfahren nach einem der Ansprüche 13 oder 14, **dadurch gekennzeichnet, dass**
in Schritt a) der Kolben (2, 52) mit einem Mischstab (9, 59) in Richtung der Rückseite der Kartusche (1, 51) gedrückt wird, wobei der Mischstab (9, 59) beweglich in einer gasdichten Durchführung, insbesondere in der Austragsöffnung, im Kartuschenkopf (3, 53) gelagert ist, und in Schritt c) die Mischung der Monomerflüssigkeit (42, 92) mit der ersten Ausgangskomponente (4, 54) durch Bewegen einer mit dem Mischstab (9, 59) verbundenen Mischeinrichtung (14, 64) im Innenraum der Kartusche (1, 51) zwischen dem Kolben (2, 52) und dem Kartuschenkopf (3, 53) erfolgt, indem der Mischstab (9, 59) und damit die Mischeinrichtung (14, 64) bewegt wird, wobei vorzugsweise zwischen Schritt c) und d) die Mischeinrichtung (14, 64) mit dem Mischstab (9, 59) zum Kartuschenkopf (3, 53) gezogen wird und ein Kern (10, 60) aus dem Mischstab (9, 59) entfernt wird, so dass der Mischstab (9, 59) ohne den Kern (10, 60) ein Austragsrohr (9, 59) bildet, durch das der gemischte Knochenzementteig (44, 94) in Schritt d) aus dem Innenraum der Kartusche (1, 51) ausgepresst wird.

16. Verfahren nach einem der Ansprüche 13 bis 15, **dadurch gekennzeichnet, dass** vor Schritt d) die Kartusche (1, 51) von der Lager- und Mischvorrichtung getrennt wird und in eine Auspressvorrichtung eingesetzt wird, mit der in Schritt d) der Kolben (2, 52) mittels eines Stößels oder einer vortreibbaren Stange in Richtung des Kartuschenkopfs (3, 53) gedrückt wird, um den Knochenzementteig (44, 94) aus dem Innenraum der Kartusche (1, 51) auszutreiben, und/oder
die Aufnahme (16, 66) ein Hohlzylinder (67) ist und die Ausdrückvorrichtung (15, 65) ein Pumpkolben (15, 65) ist, der in Längsrichtung in dem Hohlzylinder (67) beweglich angeordnet ist, wobei der Pumpkolben (15, 65) durch Aufdrücken des Kolbens (2, 52) im Hohlzylinder (67) bewegt wird oder durch einen zwischen der Rückseite der Kartusche (1, 51) und dem Kolben (2, 52) im Innenraum erzeugten Überdrück oder Druck pneumatisch oder hydraulisch bewegt wird, wobei vorzugsweise der Überdruck durch eine Druckgasleitung (69) in den Hohlzylinder (67) geleitet wird oder der Druck durch eine Hydraulikleitung (69) in den Hohlzylinder (67) geleitet wird, wobei vor den Schritten a) und b) ein Monomerflüssigkeitsbehälter (8, 58), insbesondere die Glasampulle (8, 58) oder der Folienbeutel, in der Lager- und Mischvorrichtung geöffnet wird, wobei die Monomerflüssigkeit (42, 92) aus dem Monomerflüssigkeitsbehälter (8, 58) ausläuft und in die Aufnahme (16, 66) fließt, vorzugsweise durch ein Sieb (28, 78) und/oder einen Filter (28, 78) in die Aufnahme (16, 66) fließt.

## Claims

1. Storage and mixing device for two-component polymethylmethacrylate bone cements, the storage and mixing device comprising
a cartridge (1, 51) with a cylindrical internal space, whereby the cylindrical internal space is bounded on a front side by a cartridge head (3, 53) with a closed dispensing opening;
a plunger (2, 52) that is arranged such as to be axially mobile in the cylindrical internal space of the cartridge (1, 51) and that is situated at a distance from the cartridge head (3, 53), whereby the plunger touches circumferentially against the internal wall of the internal space such that the plunger (2, 52) subdivides the internal space of the cartridge (1, 51) into two areas;
a powdered first starting component (4, 54) of the bone cement that is contained in the internal space between the plunger (2, 52) and the cartridge head (3, 53);
a feed-through (5, 55) that is arranged in the cartridge head (3, 53) or in the cylinder jacket of the cartridge (1, 51) between the plunger (2, 52) and the cartridge head (3, 53), whereby the feed-through (5, 55) is connected to a fluid conduit (6, 56);
a receptacle (16, 66) for a monomer liquid (42, 92), whereby the fluid conduit (6, 56) connects the feed-through (5, 55) to the receptacle (16, 66) in a liquid-permeable manner, whereby the monomer liquid (42, 92) is contained in the receptacle (16, 66) or can be filled or guided into the receptacle (16, 66) as second starting component of the bone cement;
a dispensing device (15, 65) that can be pushed into the receptacle (16, 66) such that monomer liquid (42, 92) can be pushed from the receptacle (16, 66) into the fluid conduit (6, 56),
**characterised in that**
the plunger (2, 52) is situated at a distance from a rear side of the cylindrical internal space that is opposite from the front side such that a motion of the plunger (2, 52) in the direction of the rear side can push the dispensing device (15, 65) into the receptacle (16, 66) and can generate a negative pressure in the internal space of the cartridge (1, 51) between the plunger (2, 52) and the cartridge head (3, 53).

2. Storage and mixing device according to claim 1, **characterised in that** the plunger (2, 52) can be pushed or drawn manually in the direction of the rear side of the internal space by means of a rod (9, 59) or by any other force transmitting means, whereby the rod (9, 59) or the force transmitting means preferably has an actuation means or a handle (12, 62) for operation of the rod (9, 59) or of the force transmitting means attached to it.

3. Storage and mixing device according to claim 1 or 2, **characterised in that** a filter (36, 86), in particular a pore filter (36, 86), is arranged in the cartridge head (3, 53), in the feed-through (5, 55) and/or in the fluid conduit (6, 56), whereby the filter (36, 86) is permeable to the monomer liquid (42, 92) and is impermeable to the powdered first starting component (4, 54) and/or
the plunger (2, 52) is situated at an appropriate distance from a rear side of the cylindrical internal space that is situated opposite from the front side such that the negative pressure can be used to aspirate the monomer liquid (42, 92) from the fluid conduit (6, 56) into the internal space of the cartridge (1, 51).

4. Storage and mixing device according to any one of the preceding claims, **characterised in that**
the storage and mixing device comprises a container (7, 57) that is separate from the cartridge (1, 51) and from the receptacle (16, 66) and which contains the monomer liquid (42, 92), whereby the container (7, 57) is connected to the receptacle (16, 66), in particular is connected by means of an opening (30, 80), whereby, preferably, a closed glass ampoule (8, 58) or a film pouch containing the monomer liquid (42, 92) is or can be arranged in the container (7, 57), whereby the glass ampoule (8, 58) can be broken open inside the container (7, 57) or the film pouch can be punctured or torn open inside the container (7, 57).

5. Storage and mixing device according to claim 4, **characterised in that** the cartridge (1, 51), the receptacle (16, 66), the separate container (7, 57), and the fluid conduit (6, 56) are connected to a common stand (20, 70), whereby the receptacle (16, 66), the container (7, 57), and the fluid conduit (6, 56) are connected to the stand (20, 70) such as to be fixed to it, and the cartridge (1, 51) is connected to the stand (20, 70) such as to be detachable, preferably the cartridge (1, 51) is screwed to the stand (20, 70) by means of a thread or is connected to the stand (20, 70) by means of a snap-in connection (22, 23, 72, 73), and/or the separate container (7, 57) contains a monomer liquid container (8, 58), in particular contains a film pouch or a glass ampoule (8, 58), that can be opened inside the separate container (7, 57) such that the monomer liquid (42, 92) flows from the opened monomer liquid container (8, 58) into the receptacle (16, 66), whereby the container (7, 57) preferably has an externally operable opening facility for opening of the monomer liquid container (8, 58) arranged on it.

6. Storage and mixing device according to any one of the preceding claims, **characterised in that**
the internal space of the cartridge (1, 51) between the plunger (2, 52) and the cartridge head (3, 53) has a mixing facility (14, 64) arranged in it, by means of which the first starting component (4, 54) can be mixed with the monomer liquid (42, 92) in the internal space of the cartridge (1, 51).

7. Storage and mixing device according to claim 6, **characterised in that** the mixing facility (14, 64) can be operated from outside the cartridge (1, 51) by means of a mixing rod (9, 59), whereby the mixing rod is guided through the cartridge head (3, 53) such as to be gas-tight, and is rotatable and is mobile in axial direction, preferably with the mixing rod (9, 59) being guided through the dispensing opening, whereby the plunger (2, 52) preferably can be pushed in the direction of the rear side of the internal space of the cartridge (1, 51) by means of the mixing rod (9, 59) and/or the mixing rod (9, 59) is a dispensing tube (9, 59), in which a manually removable core (10, 60) is arranged such that the mixed bone cement dough (44, 94) can be expelled from the internal space of the cartridge (1, 51) through the dispensing tube (9, 59) without the core (10, 60).

8. Storage and mixing device according to any one of the preceding claims,
**characterised in that**
a limit stop (18, 68) is provided on the rear side in the internal space of the cartridge (1, 51) and limits the motion of the plunger (2, 52) in the direction of the rear side, and/or
the plunger (2, 52) possesses at least one snap-in element that can be snapped into an opposite snap-in element on the inside of the cartridge (1, 51) on the rear side of the internal space in detachable manner.

9. Storage and mixing device according to any one of the preceding claims,
**characterised in that**
a gas-permeable opening, in particular a closable gas-permeable opening, is arranged in the cartridge head (3, 53), whereby a porous disk (36, 86), which is permeable to gases and impermeable to solid particles, is arranged between the first starting component (4, 54) and the opening, whereby the porous disk (36, 86) is preferably arranged in the cartridge head (3, 53).

10. Storage and mixing device according to any one of the preceding claims,
**characterised in that**
a merging site (30, 80) for supply of the monomer liquid (42, 92) into the receptacle (16, 66) is arranged in the area of the dispensing device (15, 65), whereby the merging site (30, 80) is being closed by the dispensing device (15, 65) being pushed in at the beginning of the motion of the dispensing device (15, 65) into the receptacle (16, 66).

11. Storage and mixing device according to any one of the preceding claims,
**characterised in that**
the receptacle (16) is arranged on the rear side of the cartridge (1) and the plunger (2) moving in the direction of the rear side of the cartridge (1) pushes the dispensing device (15) into the receptacle (16), whereby, preferably, the area between the dispensing device (15 and the plunger (2) is open toward the outside, whereby, preferably, the plunger (2) is situated at a distance from the dispensing device (15) such that a motion of the plunger (2) in the direction of the rear side of the cartridge (1) initially builds up a negative pressure in the internal space of the cartridge (1) in a first section, without the dispensing device (15) being moved, and, in addition, the dispensing device (15) is being pushed into the receptacle (16) in a second section,
and/or
the dispensing device (15) is a pump plunger (15) and the receptacle (16) is a hollow cylinder, in which the pump plunger (15) is axially mobile, whereby an extension, in particular in the form of a cylinder-shaped body, is arranged on the top side of the pump plunger (15) and has an external diameter that is smaller than or equal to the internal diameter of the internal space of the cartridge (1), whereby the pump plunger (15) with the extension is appropriately arranged in the hollow cylinder such that the extension projects into the internal space of the cartridge (1).

12. Storage and mixing device according to any one of the claims 1 to 10,
**characterised in that**
the receptacle (66) is bounded by a closed hollow cylinder (67) that is separate from the cartridge (51) and in which a pump plunger (65), as dispensing device (65), is supported such as to be axially mobile, whereby the pump plunger (65) closes against the internal wall of the hollow cylinder (67) both circumferentially and tightly, whereby the hollow cylinder (67) is connected to the fluid conduit (56) at a first, preferably bottom, base surface, and is connected to the internal space of the cartridge (51) at a second, preferably top, base surface by means of a compressed gas conduit (69) or a hydraulic conduit (69) on the rear side of the cartridge (51), whereby the rear side of the cartridge (51) is closed except for the merging site into the compressed gas conduit (69) or the hydraulic conduit (69), such that, when the plunger (52) moves in the direction of the rear side of the internal space, an overpressure or a pressure is generated in the space between the plunger (52) and the rear side and is guided through the compressed gas conduit (69) or the hydraulic conduit (69) into the hollow cylinder (67).

13. Method for the mixing of the starting components (4, 42, 54, 92) of a bone cement, in particular of a two-component polymethylmethacrylate bone cement, with a storage and mixing device according to at least one of the preceding claims, comprising the following steps of
a) moving the plunger (2, 52) in the direction of the rear side of the cylindrical internal space of the cartridge (1, 51), whereby the motion of the plunger (2, 52) generates a negative pressure in the internal space of the cartridge (1, 51) between the plunger (2, 52) and the cartridge head (3, 53);
b) the motion of the plunger (2, 52) pushes the dispensing device (15, 65) into the receptacle (16, 66), whereby the motion of the dispensing device (15, 65) pushes a monomer liquid (42, 92) that is contained in the receptacle (16, 66) through the fluid conduit (6, 56) into the internal space of the cartridge (1, 51);
c) mixing the monomer liquid (42, 92) and the first starting component (4, 54) in the internal space of the cartridge (1, 51) to form the bone cement dough (44, 94); and
d) expelling the bone cement dough (44, 94) by propelling the plunger (2, 52) in the direction of the cartridge head (3, 53) out of the internal space of the cartridge (1, 51).

14. Method according to claim 13, **characterised in that**
the monomer liquid (42, 92) is aspirated from the fluid conduit (6, 56) into the internal space of the cartridge (1, 51) by the negative pressure in the internal space of the cartridge 1, 51) and/or
ahead of step a), the monomer liquid (42, 92) is guided into the receptacle (16, 66), in particular after opening a monomer liquid container (8, 58) in a container (7, 57) of the storage and mixing device.

15. Method according to anyone of the claims 13 or 14, **characterised in that** in step a), the plunger (2, 52) is pushed in the direction of the rear side of the cartridge (1, 51) by means of a mixing rod (9, 59), whereby the mixing rod (9, 59) is supported such as to be mobile in a gas-tight feed-through, in particular in the dispensing opening, in the cartridge head (3, 53), and in step c), the mixing of the monomer liquid (42, 92) with the first starting component (4, 54) takes place by moving a mixing facility (14, 64), which is connected to the mixing rod (9, 59), in the internal space of the cartridge (1, 51) between the plunger (2, 52) and the cartridge head (3, 53) by moving the mixing rod (9, 59) and therefore the mixing facility (14, 64), whereby, preferably between steps c) and d), the mixing facility (14, 64) is pulled toward the cartridge head (3, 53) by means of the mixing rod (9, 59) and a core (10, 60) is removed from the mixing rod (9, 59) such that the mixing rod (9, 59) without the core (10, 60) forms a dispensing tube (9, 59) through which the mixed bone cement dough (44, 94) is extruded from the internal space of the cartridge (1, 51) in step d).

16. Method according to any one of the claims 13 to 15, **characterised in that** ahead of step d), the cartridge (1, 51) is being separated from the storage and mixing device and is being inserted into a dispensing device by means of which the plunger (2, 52) is pushed in the direction of the cartridge head (3, 53) in step d) by means of a pestle or a rod that can be propelled in order to expel the bone cement dough (44, 94) from the internal space of the cartridge (1, 51), and/or
the receptacle (16, 66) is a hollow cylinder (67) and the dispensing device (15, 65) is a pump plunger (15, 65) that is arranged such as to be mobile in longitudinal direction in the hollow cylinder (67), whereby the pump plunger (15, 65) is being moved in the hollow cylinder (67) by pushing the plunger (2, 52) onto it or is being moved pneumatically or hydraulically by an overpressure or pressure that is generated in the internal space between the rear side of the cartridge (1, 51) and the plunger (2, 52), whereby the overpressure is preferably guided through a compressed gas conduit (69) into the hollow cylinder (67) or the pressure is guided through a hydraulic conduit (69) into the hollow cylinder (67), whereby
ahead of steps a) and b), a monomer liquid container (8, 58), in particular the glass ampoule (8, 58) or the film pouch, is being opened in the storage and mixing device, whereby the monomer liquid (42, 92) leaks from the monomer liquid container (8, 58) and flows into the receptacle (16, 66), preferably flows into the receptacle (16, 66) through a sieve (28, 78) and/or a filter (28, 78).

## Revendications

1. Dispositif de stockage et de mélange pour ciments osseux à deux composants à base de polyméthacrylate de méthyle, le dispositif de stockage et de mélange comportant
une cartouche (1, 51) avec un espace intérieur cylindrique, l'espace intérieur cylindrique étant limité sur une face frontale par une tête de cartouche (3, 53) avec une ouverture de sortie fermée,
un piston (2, 52), qui est agencé avec une mobilité axiale dans l'espace intérieur cylindrique de la cartouche (1, 51) et qui est distant de la tête de cartouche (3, 53), le piston (2, 52) étant sur sa périphérie en appui au niveau de la paroi intérieure de l'espace intérieur, de sorte que le piston (2, 52) sépare l'espace intérieur de la cartouche (1, 51) en deux zones,
un premier composant de départ (4, 54) pulvérulent du ciment osseux qui est contenu dans l'espace intérieur entre le piston (2, 52) et la tête de cartouche (3, 53),
un passage (5, 55), qui est agencé dans la tête de cartouche (3, 53) ou dans l'enveloppe cylindrique de la cartouche (1, 51) entre le piston (2, 52) et la tête de cartouche (3, 53), le passage (5, 55) étant relié à une conduite de fluide (6, 56),
un logement (16, 66) pour un monomère liquide (42, 92), la conduite de fluide (6, 56) reliant de manière perméable aux liquides le passage (5, 55) au logement (16, 66), le monomère liquide (42, 92) étant en tant que deuxième composant de départ du ciment osseux contenu dans le logement (16, 66) ou pouvant être rempli ou acheminé en tant que deuxième composant de départ du ciment osseux dans le logement (16, 66),
un dispositif d'extraction (15, 65), pouvant être poussé dans le logement (16, 66), de sorte que le monomère liquide (42, 92) puisse être pressé hors du logement (16, 66) dans la conduite de fluide (6, 56),
**caractérisé en ce, que**
le piston (2, 52) soit distant d'une face arrière opposée à la face frontale de l'espace intérieur cylindrique, de sorte qu'un mouvement du piston (2, 52) dans le sens vers la face arrière permette de pousser le dispositif d'extraction (15, 65) dans le logement (16, 66) et de créer une dépression dans l'espace intérieur de la cartouche (1, 51) entre le piston (2, 52) et la tête de cartouche (3, 53).

2. Dispositif de stockage et de mélange selon la revendication 1, **caractérisé en ce, que** le piston (2, 52) puisse être poussé ou tiré manuellement dans l'espace intérieur de la cartouche (1, 51) dans le sens vers la face arrière de l'espace intérieur avec une barre (9, 59) ou avec un autre moyen de transmission de force, un moyen d'actionnement ou une poignée (12, 62) étant de préférence fixé à la barre (9, 59) ou au moyen de transmission de force pour actionner la barre (9, 59) ou le moyen de transmission de force.

3. Dispositif de stockage et de mélange selon la revendication 1 ou 2, **caractérisé en ce, que**
soit agencé dans la tête de cartouche (3, 53), dans le passage (5, 55) et/ou dans la conduite de fluide (6, 56) un filtre (36, 86), en particulier un filtre poreux (36, 86), le filtre (36, 86) étant perméable pour le monomère liquide (42, 92) et imperméable pour le premier composant de départ (4, 54) pulvérulent et/ou
que le piston (2, 52) soit éloigné d'une telle distance d'une face arrière opposée à la face frontale de l'espace intérieur cylindrique, de sorte qu'avec la dépression, le monomère liquide (42, 92) puisse être aspiré hors de la conduite de fluide (6, 56) dans l'espace intérieur de la cartouche (1, 51).

4. Dispositif de stockage et de mélange selon l'une des revendications précédentes, **caractérisé en ce, que**
le dispositif de stockage et de mélange comporte en position séparée de la cartouche (1, 51) et du logement (16, 66) un récipient (7, 57), qui contient le monomère liquide (42, 92), le récipient (7, 57) étant relié au logement (16, 66), en particulier par un orifice (30, 80), une ampoule en verre (8, 58) fermée ou un sac en plastique contenant le monomère liquide (42, 92) étant de préférence agencé ou devant de préférence être agencé dans le récipient (7, 57), l'ampoule en verre (8, 58) pouvant être rompue à l'intérieur du récipient (7, 57) ou le sac en plastique pouvant être percé ou déchiré à l'intérieur du récipient (7, 57).

5. Dispositif de stockage et de mélange selon la revendication 4, **caractérisé en ce, que**
la cartouche (1, 51), le logement (16, 66), le récipient séparé (7, 57) et la conduite de fluide (6, 56) soient reliés ensemble avec un pied-support (20, 70) commun, le logement (16, 66), le récipient (7, 57) et la conduite de fluide (6, 56) étant reliés de manière fixe avec le pied-support (20, 70) et la cartouche (1, 51) étant reliée de manière libérable au pied-support (20, 70), de préférence que la cartouche (1, 51) soit vissée au pied-support (20, 70) au moyen d'un filetage ou qu'elle soit reliée au pied-support (20, 70) via un crantage (22, 23, 72, 73) et/ou
que le récipient séparé (7, 57) contienne un réservoir de monomère liquide (8, 58), notamment un sac en plastique ou une ampoule en verre (8, 58), qui doit être ouvert au sein du récipient séparé (7, 57), de sorte que le monomère liquide (42, 92) coule du réservoir de monomère liquide (8, 58) dans le logement (16, 66), un dispositif d'ouverture manoeuvrable de l'extérieur pour ouvrir le réservoir de monomère liquide (8, 58) étant de préférence agencé au récipient (7, 57).

6. Dispositif de stockage et de mélange selon l'une des revendications précédentes, **caractérisé en ce, que**
soit agencé dans l'espace intérieur de la cartouche (1, 51) entre le piston (2, 52) et la tête de cartouche (3, 53) un dispositif de mélange (14, 64), qui est destiné à servir pour mélanger le premier composant de départ (4, 54) et le monomère liquide (42, 92) dans l'espace intérieur de la cartouche (1, 51).

7. Dispositif de stockage et de mélange selon la revendication 6, **caractérisé en ce, que**
le dispositif de mélange (14, 64) soit manoeuvrable par l'intermédiaire d'une barre de mélange (9, 59) de l'extérieur de la cartouche (1, 51), la barre de mélange (9, 59) étant guidée de manière étanche aux gaz au travers de la tête de cartouche (3, 53) avec une mobilité en rotation et axiale, la barre de mélange (9, 59) étant de préférence guidée au travers de l'ouverture de sortie, le piston (2, 52) devant de préférence être poussé par l'intermédiaire de la barre de mélange (9, 59) dans le sens de la face arrière de l'espace intérieur de la cartouche (1, 51) et/ou la barre de mélange (9, 59) étant un tube d'extraction (9, 59), dans lequel est agencé un noyau (10, 60) amovible manuellement, de sorte que le mélange de pâte de ciment osseux (44, 94) puisse être extrait sans le noyau (10, 60) de l'espace intérieur de la cartouche (1, 51) au travers du tube d'extraction (9, 59).

8. Dispositif de stockage et de mélange selon l'une des revendications précédentes, **caractérisé en ce, que**
soit prévu dans l'espace intérieur de la cartouche (1, 51) au niveau de la face arrière un butoir (18, 68), qui limite le mouvement du piston (2, 52) dans le sens vers la face arrière, et/ou
que le piston (2, 52) possède au moins un élément de crantage, qui peut s'encranter de manière libérable dans un contre-crantage au niveau de la face intérieure de la cartouche (1, 51) dans la zone de la face arrière de l'espace intérieur.

9. Dispositif de stockage et de mélange selon l'une des revendications précédentes, **caractérisé en ce, que**
soit agencé dans la tête de cartouche (3, 53) un orifice perméable aux gaz, notamment un orifice perméable aux gaz pouvant être fermé, un disque poreux (36, 86) perméable aux gaz et imperméable aux particules solides étant agencé entre le premier composant de départ (4, 54) et l'orifice, le disque poreux (36, 86) étant de préférence agencé dans la tête de cartouche (3, 53).

10. Dispositif de stockage et de mélange selon l'une des revendications précédentes, **caractérisé en ce, que**
soit agencée dans la zone du dispositif d'extraction (15, 65) une embouchure (30, 80) pour introduire le monomère liquide (42, 92) dans le logement (16, 66), l'embouchure (30, 80) se fermant par la poussée du dispositif d'extraction (15, 65) au début du mouvement du dispositif d'extraction (15, 65) dans le logement (16, 66).

11. Dispositif de stockage et de mélange selon l'une des revendications précédentes, **caractérisé en ce, que**
le logement (16) soit agencé au niveau de la face arrière de la cartouche (1) et que le piston (2) pousse le dispositif d'extraction (15) dans le logement (16) pendant le mouvement dans le sens vers la face arrière de la cartouche (1), la zone entre le dispositif d'extraction (15) et le piston (2) étant de préférence ouverte vers l'extérieur, le piston (2) étant de préférence distant du dispositif d'extraction (15), de sorte que lors d'un mouvement du piston (2) dans le sens vers la face arrière de la cartouche (1) une dépression dans l'espace intérieur de la cartouche (1) soit d'abord générée sur une première section, sans que le dispositif d'extraction (15) soit mis en mouvement, et que, en outre, le dispositif d'extraction (15) soit poussé dans le logement (16) sur une deuxième section, et/ou
que le dispositif d'extraction (15) soit un piston de pompage (15) et que le logement (16) soit un cylindre creux, dans lequel le piston de pompage (15) soit mobile axialement, une rallonge étant agencée sur la partie supérieure du piston de pompage (15), en particulier sous la forme d'un corps cylindrique, ladite rallonge ayant un diamètre extérieur inférieur ou égal au diamètre intérieur de l'espace intérieur de la cartouche (1), le piston de pompage (15) avec la rallonge étant agencé dans le cylindre creux de sorte, que la rallonge pénètre dans l'espace intérieur de la cartouche (1).

12. Dispositif de stockage et de mélange selon l'une des revendications 1 à 10,
**caractérisé en ce, que**
le logement (66) soit limité par un cylindre creux (67) fermé séparé de la cartouche (51), dans lequel un piston de pompage (65) est monté avec une mobilité axiale en tant que dispositif d'extraction (65), le piston de pompage (65) étant en contact de manière étanche sur toute sa périphérie avec la paroi intérieure du cylindre creux (67), le cylindre creux (67) étant relié avec une première surface de base, de préférence inférieure, avec la conduite de fluide (56) et avec une deuxième surface de base, de préférence supérieure, via une conduite de gaz sous pression (69) ou une conduite hydraulique (69) à la face arrière de la cartouche (51) avec l'espace intérieur de la cartouche (51), la face arrière de la cartouche (51) étant fermée jusqu'à l'embouchure dans la conduite de gaz sous pression (69) ou conduite hydraulique (69), de sorte qu'un mouvement du piston (52) dans le sens vers la face arrière de l'espace intérieur génère dans l'espace entre le piston (52) et la face arrière une surpression ou une pression, qui est transférée dans le cylindre creux (67) au travers de la conduite de gaz sous pression (69) ou de la conduite hydraulique (69).

13. Procédé de mélange des composants de départ (4, 42, 54, 92) d'un ciment osseux, en particulier d'un ciment osseux à deux composants à base de polyméthacrylate de méthyle, avec un dispositif de stockage et de mélange selon au moins une des revendications précédentes, comprenant les étapes suivantes,
a) le piston (2, 52) est mis en mouvement dans le sens vers la face arrière de l'espace intérieur cylindrique de la cartouche (1, 51), de sorte que le mouvement du piston (2, 52) dans l'espace intérieur de la cartouche (1, 51) génère une dépression entre le piston (2, 52) et la tête de cartouche (3, 53),
b) par le mouvement du piston (2, 52), le dispositif d'extraction (15, 65) est poussé dans le logement (16, 66), ce qui provoque par le mouvement du dispositif d'extraction (15, 65) la poussée d'un monomère liquide (42, 92) contenu dans le logement (16, 66) au travers de la conduite de fluide (6, 56) dans l'espace intérieur de la cartouche (1, 51),
c) le monomère liquide (42, 92) et le premier composant de départ (4, 54) sont mélangés dans l'espace intérieur de la cartouche (1, 51) pour former la pâte de ciment osseux (44, 94), et
d) la pâte de ciment osseux (44, 94) est extraite de l'espace intérieur de la cartouche (1, 51) par l'avancement du piston (2, 52) dans le sens vers la tête de cartouche (3, 53).

14. Procédé selon la revendication 13, **caractérisé en ce, que** le monomère liquide (42, 92) soit aspiré par la dépression dans l'espace intérieur de la cartouche (1, 51) hors de la conduite de fluide (6, 56) dans l'espace intérieur de la cartouche (1, 51) et/ou
qu'avant l'étape a), le monomère liquide (42, 92) soit introduit dans le logement (16, 66), notamment après l'ouverture d'un réservoir de monomère liquide (8, 58) dans un récipient (7, 57) du dispositif de stockage et de mélange.

15. Procédé selon l'une des revendications 13 ou 14, **caractérisé en ce, que** dans l'étape a), le piston (2, 52) soit poussé avec une barre de mélange (9, 59) dans le sens vers la face arrière de la cartouche (1, 51), la barre de mélange (9, 59) étant montée de manière mobile dans un passage étanche aux gaz, en particulier dans l'ouverture de sortie, dans la tête de cartouche (3, 53), et que dans l'étape c) soit exécuté dans l'espace intérieur de la cartouche (1, 51) entre le piston (2, 52) et la tête de cartouche (3, 53) le mélange du monomère liquide (42, 92) avec le premier composant de départ (4, 54) par le mouvement d'un dispositif de mélange (14, 64) relié à la barre de mélange (9, 59), par la mise en mouvement de la barre de mélange (9, 59) et, en conséquence, du dispositif de mélange (14, 64), et que de préférence entre l'étape c) et d), le dispositif de mélange (14, 64) soit tiré avec la barre de mélange (9, 59) vers la tête de cartouche (3, 53) et qu'un noyau (10, 60) soit retiré de la barre de mélange (9, 59), de sorte que la barre de mélange (9, 59) sans le noyau (10, 60) forme un tube d'extraction (9, 59), au travers duquel, dans l'étape d), la pâte de ciment osseux (44, 94) mélangée est pressée hors de l'espace intérieur de la cartouche (1, 51).

16. Procédé selon l'une des revendications 13 à 15, **caractérisé en ce, que** avant l'étape d), la cartouche (1, 51) soit séparée du dispositif de stockage et de mélange et soit introduite dans un dispositif d'extraction, au moyen duquel le piston (2, 52) est poussé dans l'étape d), par l'intermédiaire d'une tige métallique (steel) ou d'une barre pouvant être avancée, dans le sens vers la tête de cartouche (3, 53), pour extraire la pâte de ciment osseux (44, 94) de l'espace intérieur de la cartouche (1, 51), et/ou
que le logement (16, 66) soit un cylindre creux (67) et que le dispositif d'extraction (15, 65) soit un piston de pompage (15, 65), qui est agencé de manière mobile dans le sens longitudinal dans le cylindre creux (67), le piston de pompage (15, 65) étant mis en mouvement par le pressage du piston (2, 52) dans le cylindre creux (67) ou, de manière pneumatique ou hydraulique, par une surpression ou une pression générée entre la face arrière de la cartouche (1, 51) et le piston (2, 52) dans l'espace intérieur, la surpression étant de préférence transférée dans le cylindre creux (67) au travers d'une conduite de gaz sous pression (69) ou la pression étant de préférence transférée dans le cylindre creux (67) via une conduite hydraulique (69), et que
avant les étapes a) et b), soit ouvert dans le dispositif de stockage et de mélange un réservoir de monomère liquide (8, 58), en particulier l'ampoule en verre (8, 58) ou le sac en plastique, de sorte que le monomère liquide (42, 92) s'écoule hors du réservoir de monomère liquide (8, 58) et coule dans le logement (16, 66), qu'il coule de préférence dans le logement (16, 66) à travers une crépine (28, 78) et/ou un filtre (28, 78).
